# EUROPEAN PATENT APPLICATION

(11) **EP 4 614 501 A1**
(43) Date of publication of application: **10.09.2025**
(21) Application number: 24162030.1
(22) Date of filing: 07.03.2024
(51) Int. Cl.: G16H 10/40

(54) **ANALYTICAL SAMPLE CONTAINER CLASSIFICATION**

(71) Applicant: Roche Diagnostics International AG, 6343 Rotkreuz (CH)
(72) Inventor: GLAUSER, Michael, 6343 Rotkreuz ZG (CH); GUTMANN, Oliver, 6343 Rotkreuz ZG (CH); JANNER-SCHUBIGER, Gabriele Piero, 6343 Rotkreuz ZG (CH); SAROFIM, Emad, 6343 Rotkreuz ZG (CH); SCHWEIGHAUSER, Stephan, 6343 Rotkreuz ZG (CH)
(74) Representative: Peterreins Schley

(57) **Abstract**

A computer implemented method (80) for analytical sample container classification, wherein the method comprises:
- obtaining (82) a digital representation of a visual identifier (3A) associated with a sample container (1);
- identifying (84) at least one apparatus (20PRE-1) comprised within an analytical system (20), wherein the analytical system (20) is intended to perform at least one analytical test using the sample container (1), wherein the at least one apparatus (20PRE-1) comprises an optical identifier reader;
- classifying (86) the sample container (1) associated with the visual identifier (3A) by characterizing the ability of the optical identifier reader of the at least one apparatus (20PRE-1) comprised in the analytical system (20) to decode the visual identifier (3A) associated with the sample container (1), to thereby generate a corresponding classification result characterizing the sample container (1) associated with the visual identifier (3A); and
- outputting (88) a message defining the classification result.

## Description

### Technical Field

This disclosure relates to a computer implemented method for analytical sample container classification, and an associated system, apparatus, computer implemented method for training a classifier of analytical sample containers comprising visual identifiers, computer program element, and a computer program product.

### Background

An in vitro diagnostic (IVD) analysis laboratory is capable of analysing a large number of patient samples. Each sample is isolated in an analytical sample container prior to entry into the IVD laboratory. A sample can potentially comprise at least one analyte of interest, e.g. molecules, ions, proteins, metabolites, pathogens, and the like. It is typically one of the tasks of IVD testing to detect the presence, absence and/or a concentration of one or more analytes in a sample. More generically, IVD testing can refer to determining a biological property of a sample. IVD testing can comprise performing at least one analytical test on a sample, wherein the analytical test can allow conclusions about the biological properties of the sample to be drawn. The analytical test can e.g. comprise adding a reagent to the sample, a possible detectable reaction of the sample with the reagent, and/or a detecting or nondetection of this reaction. Detecting of a reaction can e.g. comprise measuring a physical value of the sample (resp. a composite obtained by using the sample such as a sample-reagent mixture), such as a spectrum and/or an intensity of a radiation reflected by and/or transmitted through the sample (resp. the composite obtained by using the sample).

IVD laboratories are complex and custom-designed based on requirements of each laboratory. The number of analytical sample containers processed by a typical IVD laboratory per day may range in the thousands or tens of thousands. The identification of analytical sample containers can, thus, only be effectively carried out using automated means such as attaching visual identifiers such as barcodes or QR codes to sample containers. Although barcodes and QR codes have error correcting measures, they are still susceptible to incorrect application or damage during transport. When a sample container having a damaged visual identifier enters an IVD laboratory, a range of errors can occur which can only be manually addressed. Therefore, sample container identification approaches can be improved further.

### Summary

According to a first aspect, there is provided a computer implemented method for analytical sample container classification. The method comprises:
- obtaining a digital representation of a visual identifier associated with a sample container;
- identifying at least one apparatus comprised within an analytical system, wherein the analytical system is intended to perform at least one analytical test using the sample container, wherein the at least one apparatus comprises an optical identifier reader and/or camera;
- classifying the sample container associated with the visual identifier by characterizing the ability of the optical identifier reader and/or camera of the at least one apparatus comprised in the analytical system to read the visual identifier associated with the sample container, to thereby generate a corresponding classification result characterizing the sample container associated with the visual identifier; and
- outputting a message defining the classification result.

An effect is that a sample container associated with the visual identifier can be assessed prior to admission into an analytical system for imperfections or problems with the visual identifier used to identify the sample container. An analytical system may comprise a range of analytical equipment, and before performing a test as part of a workflow on a sample container, the sample container needs to be identified. If the visual identifier is damaged, badly printed, or incorrectly applied to the sample container, there is a risk that the visual identifier cannot be correctly decoded by at least one apparatus comprised within the analytical system.

Presently, significant productivity problems occur owing to the admission of sample containers into an analytical system, when a sample container bears a visual identifier that cannot be decoded by at least one apparatus comprised within an analytical system required to perform a related test order. Presently, the best case scenario is that the unidentifiable sample container is rerouted within the analytical system to a holding station where trained operators of the analytical system can manually inspect the sample container and provide a new visual identifier. However, the process of rerouting the unidentifiable sample container through the transport subsystem of the analytical system may, in itself, cause inefficiencies in other workflows that are being executed by the analytical system. If the test associated with the unidentifiable sample container is a STAT test, in other words an extremely urgent test, the time incurred in addressing the rerouted unidentifiable sample container could cause the result of the STAT test to be too late for use. Furthermore, a large system could incur so many inefficiencies owing to damage or unidentifiable visual identifiers that large numbers of extra staff are needed to address unidentifiable sample containers. In a worst-case scenario, the presence of an unidentifiable sample container at an analytical apparatus within the analytical system could risk the entire analytical system needing to be paused whilst the unidentifiable sample container is removed.

Accordingly, performing analytical sample container classification according to the first aspect enables the visual identifiers associated with each sample container to be proactively compared with the capabilities of one, or more, analytical apparatuses comprised within an analytical system that the sample container is being sent to.

In an example, a defective visual identifier can be detected at the collection site where the sample container is initially filled with a sample obtained from a patient. The collection site includes remote settings e.g. GP or home settings and a clinical site. This means that a clinician or lab technician can correct the problem with the visual identifier prior to the sample container being dispatched to a central laboratory comprising the analytical system. For example, a mobile phone application can be used to scan the sample container comprising a visual identifier, and the mobile phone application can provide feedback to a clinician on whether the selected central laboratory can process a desired test order based on a visual analysis of the visual identifier obtained, for example, by a camera of the mobile phone.

In an example, a label printer in a collection site where patient samples are obtained may be defective, and not capable of producing visual identifiers for association with a sample container to a required for a specific test order. In this case, this fact can be discovered ahead of time and the sample container can be marked for re-labelling upon arrival at the central laboratory comprising the analytical system.

In another example, a defective visual identifier can be detected upon accession of a sample into an analytical system. For example, a visual identifier associated with the sample container may be in good condition upon leaving a clinic, but may be damaged in transit.

In another example, visual identifiers may be measured at one or more points or nodes within the analytical system. For example, visual identifiers could be damaged within the central laboratory and problems caused by such damage can be detected in advance and corrected.

Prevention of problems caused by damaged visual identifiers associated with sample containers therefore lead to an increase in throughput of the overall analytical system, or a reduction in the amount of downtime of the overall analytical system. On average, urgent tests are less likely to be delayed owing to downtime caused by damaged visual identifiers associated with sample containers. On average, laboratory workers will need to devote less effort to correcting the problems of damaged visual identifiers associated with sample containers once the sample containers have entered the analytical system, because sample containers bearing defective visual identifiers that may cause a problem can be rerouted prior to admission into the analytical system.

According to a second aspect, there is provided a system comprising an apparatus comprising an optical identifier reader and/or a camera configured to obtain a digital representation of an visual identifier associated with a sample container, an analytical system comprising at least one apparatus configured to perform at least one analytical test, a communications network, and a data processing agent that is communicably coupled to the apparatus and the analytical system via the communications network.

The data processing agent is configured to obtain a digital representation of a visual identifier associated with a sample container, to identify at least one apparatus comprised within an analytical system, wherein the analytical system is intended to perform at least one analytical test using the sample container.

The at least one apparatus comprises an optical identifier reader, wherein the data processing agent is further configured to classify the visual identifier associated with the sample container by characterizing the ability of the optical identifier reader and/or camera of the at least one apparatus comprised in the analytical system to read the visual identifier associated with the sample container, to thereby generate a corresponding classification result characterizing the visual identifier associated with the sample container to output a message defining the classification result.

According to a third aspect, there is provided an apparatus comprising a communications interface, a processor, and a memory interface.

The processor is configured to host a data processing agent that, in use, is communicably coupled to an apparatus and an analytical system via a communications network.

The data processing agent is configured to obtain a digital representation of a visual identifier associated with a sample container, and to identify at least one apparatus comprised within the analytical system. The analytical system is intended to perform at least one analytical test using the sample container. The data processing agent is further configured to classify the visual identifier associated with the sample container by characterizing the ability of the optical identifier reader and/or camera of the at least one apparatus comprised in the analytical system to read the visual identifier associated with the sample container, to thereby generate a corresponding classification result characterizing the visual identifier associated with the sample container to output a message defining the classification result.

According to a fourth aspect, there is provided a computer program element comprising machine readable instructions which, when executed, performs the computer implemented method according to the first aspect.

According to a fifth aspect, there is provided a computer readable medium having encoded thereon the computer program element according to the fourth aspect.

According to a sixth aspect, there is provided a computer implemented method for training a classifier of analytical sample containers comprising visual identifiers, wherein the method comprises:
- obtaining a training set comprising a plurality of digital representations of visual identifiers associated with a corresponding plurality of sample containers;
- labelling each digital representation in the training set with a first identifier of at least one analytical test to be performed using the sample container, and optionally a second identifier of at least one analytical system to be used to perform the at least one analytical test;
- obtaining a result set defining, for each digital representation in the training set, a determination of whether, or not, the corresponding visual identifier was correctly read by all apparatuses of the at least one analytical system and
- training, using a machine learning process, a classifier using the training set and the corresponding result set.

According to a seventh aspect, is provided a computer program element comprising machine readable instructions which, when executed, performs the computer implemented method according to the fifth aspect.

According to an eighth aspect, there is provided a computer readable medium having encoded thereon the computer program element according to the seventh aspect.

According to a ninth aspect, there is provided a machine learning model comprising machine readable instructions which, when provided with an input data vector, generate an output data vector according to the classifier trained according to the sixth aspect.

Optional embodiments are defined in the dependent claims, to which the reader may now refer, and which are discussed further in this specification.

Certain terms will be used in this patent specification, the formulation of which should not be interpreted to be limited by the specific term chosen, but as to relate to the general concept behind the specific term.

As used herein, the terms "comprises," "comprising", "includes", "including", "has", "having", or any other variation thereof, are intended to cover a non-exclusive inclusion.

Certain terms will be used in this patent specification, the formulation of which relates to the general concept behind the specific term.

The reference to "at least one apparatus comprised within an analytical system" as used herein encompasses any apparatus or apparatus component operable to execute one or more processing steps / workflow steps on one or more biological samples. The expression "processing steps" thereby refers to physically executed processing steps such as centrifugation, aliquotation, sample analysis and the like. The term "apparatus comprised within an analytical system" covers pre-analytical instruments, post-analytical instruments and also analytical instruments and transport elements for transferring a sample container from a first location to a second location within an analytical system.

The expression "analytical system" as used herein encompasses any monolithic or multi-modular laboratory device comprising one or more lab-devices or operative units which are operable to execute an analytical test on one or more biological samples. The plurality of laboratory apparatuses may be operatively connected to a control unit, otherwise referred to as "laboratory middleware" or a "laboratory information system."

The term "control unit," "laboratory middleware" or "laboratory information system" as used herein encompasses any physical or virtual processing device configurable to control a laboratory system comprising a plurality of laboratory instruments in a way that workflow(s) and workflow step(s) are conducted by the laboratory system. The control unit may, for example, instruct the laboratory system (or a specific instrument thereof) to conduct pre-analytical, post analytical and analytical workflow(s)/ workflow step(s). The control unit may receive information from a data management unit regarding which steps need to be performed with a certain sample. In some embodiments, the control unit might be integral with a data management unit, may be comprised by a server computer and/or be part of one instrument or even distributed across multiple instruments of the laboratory system. The control unit may, for instance, be embodied as a programmable logic controller running a computer-readable program provided with instructions to perform operations.

The term "order" or "test order" refers to a service offered to a customer of the analytical system whereby a patient sample enters the analytical system within a sample container, a series of processing steps are performed on the patient sample, and analytical device output a result in accordance with the original test order. On some occasions, the result may be a positive or negative control and thus not associated with a patient sample. Thus, an "order" or "test order" is a user-identifiable identifier that a "control unit" parses into one, or more, workflows or workflow steps necessary to provide the eventual result.

The term "workflow" as used herein refers to a collection of workflow steps / processing steps. According to particular embodiments, the workflow defines a sequence in which the processing steps are carried out. The term "workflow step" or "processing step" as used herein encompasses any activity belonging to a workflow. The activity can be of an elementary or complex nature and is typically performed at or by one or more analytical instrument(s).

An apparatus comprised within an analytical system can comprise one or more analytical modules designed to execute respective workflows that are optimized for certain types of analysis.

The apparatus can include analytical apparatuses for one or more of clinical chemistry, immunochemistry, coagulation, haematology, etc.

Thus, the apparatus may comprise one analytical module or a combination of any of such modules with respective workflows, where pre-analytical and/or post analytical modules may be coupled to individual analytical modules or be shared by a plurality of analytical modules. Alternatively, pre-analytical and/or post-analytical functions may be performed by units integrated in an apparatus. The apparatus can comprise functional units such as liquid handling units for pipetting and/or pumping and/or mixing of samples and/or reagents and/or system fluids, and also functional units for sorting, storing, transporting, identifying, separating, and detecting.

In particular, an apparatus in an analytical system is operable to determine via various chemical, biological, physical, optical or other technical procedures a parameter value of the sample or a component thereof. An analyser may be operable to measure said parameter of the sample or of at least one analyte and return the obtained measurement value. The list of possible analysis results returned by the analyser comprises concentrations of the analyte in the sample, a digital (yes or no) result indicating the existence of the analyte in the sample (corresponding to a concentration above the detection level), optical parameters, DNA or RNA sequences, data obtained from mass spectroscopy of proteins or metabolites and physical or chemical parameters of various types. An analytical instrument may comprise units assisting with the pipetting, dosing, and mixing of samples and/or reagents. The analyser may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyser may comprise a process and detection system whose workflow is optimized for certain types of analysis. Examples of such analyser are clinical chemistry analysers, coagulation chemistry analysers, immunochemistry analysers, urine analysers, nucleic acid analysers, tissue analysers (incl. morphological strainers and histochemical stainers) used to detect the result of chemical or biological reactions or to monitor the progress of chemical or biological reactions.

The term "pre-analytical instrument"' as used herein comprises one or more lab-devices for executing one or more pre-analytical processing steps on one or more biological samples, thereby preparing the samples for one or more succeeding analytical tests. A pre-analytical processing step can be, for example, a centrifugation step, a capping-, decapping- or recapping step, an aliquotation step, a step of adding buffers to a sample and the like.

The term "post-analytical instrument" as used herein encompasses any laboratory instrument being operable to automatically process and/or store one or more biological samples. Post-analytical processing steps may comprise a recapping step, a step for unloading a sample from an analytical system or a step for transporting said sample to a storage unit or to a unit for collecting biological waste.

The term "sample" refers to a biological material suspected of containing one or more analytes of interest and whose detection, qualitative and/or quantitative, may be associated to a particular condition (e.g., a clinical condition).

The sample can be derived from any biological source, such as a physiological fluid, including, blood, saliva, ocular lens fluid, cerebral spinal fluid, sweat, urine, milk, ascites fluid, mucous, synovial fluid, peritoneal fluid, amniotic fluid, tissue, cells or the like. The sample can be pre-treated prior to use, such as preparing plasma from blood, diluting viscous fluids, lysis or the like; methods of treatment can involve filtration, centrifugation, distillation, concentration, inactivation of interfering components, and the addition of reagents. A sample may be used directly as obtained from the source in some cases or following a pre-treatment and/or sample preparation workflow to modify the character of the sample, e.g. after adding an internal standard, after being diluted with another solution or after having being mixed with reagents e.g., to enable carrying out one or more in vitro diagnostic tests, or for enriching (extracting/separating/concentrating) analytes of interest and/or for removing matrix components potentially interfering with the detection of the analyte(s) of interest.

The term "sample container" refers to any individual container for transporting, storing and/or processing a sample. In particular, said term without limitation refers to a piece of laboratory glass- or plastic-ware optionally comprising a cap on its upper end. In many cases, the sample container is a tube. The sample container is configured to receive, or be associated with a visual identifier enabling a user, and/or an apparatus, to automatically identify the sample container at any step of a workflow. In some cases, the sample container may be a planar plastic or glass slide with a mounted slice of tissue sample. In this case, the visual identifier may be a barcode, a QR code, and/or handwriting adhered to a portion of the planar plastic or glass slide that does not comprise a mounted slice of tissue sample. All forms of sample containers and with it the included sample, is identifiable, ideally uniquely, independent of its form factor.

A "network" in the present disclosure refers to a plurality of connected devices with data communication capabilities. The connected devices in a network can be designated by being managed by a particular organization. For instance, the network can be a hospital network, a laboratory network, a network of a manufacturer or a remote support engineer. In some embodiments, the network can be constituted by a set of devices which form a logical group (e.g., a network of devices of a particular organization as defined above). In addition or alternatively, the devices in a network can be located in relatively close spatial relationship (e.g., a campus, a laboratory or a hospital building). The devices of the network can be connected by a local area network. However, in other embodiments the devices of the network can be located at two or more remote locations (e.g., two different sites of a hospital).

The term "communication network" as used herein encompasses any type of wired or wireless network, including but not limited to a WIFI, GSM, UMTS or other wireless digital network or a wired network, such as Ethernet or the like. For example, the communication network may include a combination of wired and wireless networks. Analytical device status data may be transmitted over the communication network.

The term "gateway" encompasses any hardware-, firmware- and/or software- based module operable to execute program logic to allow communication with an external entity over a communications network (such as a server or another interface).

The term "server" encompasses any physical machine or virtual machine having a physical or virtual processor, capable of accepting requests from and giving responses accordingly. It shall be clear to a person of ordinary skill in the art of computer programming that the term machine may refer to a physical hardware itself, or to a virtual machine such as a JAVA Virtual Machine (JVM), or even to separate virtual machines running different Operating Systems on the same physical machine and sharing that machine's computing resources. Servers can run on any computer including dedicated computers, which individually are also often referred to as "the server" or shared resources such as virtual servers. In many cases, a computer can provide several services and have several servers running. Therefore the term server shall encompass any computerized device that shares a resource to one or more client processes. The server can receive, process, and transmit analytical device status data.

The term "user interface" encompasses any suitable piece of software and/or hardware for interactions between an operator and a machine, including but not limited to a graphical user interface for receiving as input a command from an operator and also to provide feedback and convey information thereto. Also, a system / device may expose several user interfaces to serve different kinds of users/ operators. The user interface may display user feedback about visual identifier quality and/or prompt a user to reprint a visual identifier, for example.

### Description of the Drawings

**FIG. 1** schematically illustrates two versions of a sample container having, respectively, a correctly applied visual identifier and an incorrectly applied visual identifier.
**FIG. 2** schematically illustrates four examples of barcode quality degradation.
**FIG. 3** schematically illustrates an example of a system for sample processing.
**FIG. 4** schematically illustrates a computer implemented method according to the first aspect.
**FIG. 5** schematically illustrates an example of a process for sample container examination.
**Fig. 6** schematically illustrates a data model for sample container examination.
**Fig. 7** schematically illustrates a further example of a rule-based approach for sample container processing.
**Fig. 8** schematically illustrates an example of a graphical user interface during sample container processing.
**Fig. 9** schematically illustrates an example of sample container classification by machine learning.
**Fig. 10** schematically illustrates an example of an apparatus according to the third aspect.

Note: The figures are not drawn to scale, are provided as illustration only and serve only for better understanding but not for defining the scope of the invention. No limitations of any features of the invention should be inferred from these figures.

### Detailed Description

Patient samples are typically collected outside of an analytical system (laboratory) at remote collection sites (such as medical clinics) or on the wards of a hospital. When the patient samples are collected, they are secured in a sample container (specimen tube) compatible with automated analysers present in an analytical system. The sample containers are typically prepared by personnel outside the lab such as a phlebotomist or nurse in a hospital, for example. Automated software typically provides a unique visual identifier for each sample container. The automated software may include an order entry system or a laboratory information system (LIS) typically giving unique (multiple) tube IDs as unique visual identifier(s) for an order with several tube containers. The unique visual identifier is associated with a test order for the test that the laboratory personnel intend to order for the patient. At the same time the automated software generates a new record in a management system of an operator of the analytical system, a unique visual identifier for the sample container is generated and typically supplied to a label printer. The label printer is configured to print an adhesive label as part of a print on demand process, which the laboratory personnel attaches to the sample container prior to forwarding the sample container for processing at the analytical system.

In the analytical system (laboratory) a variety of pre-analytical, analytical, post analytical, and transport means are used in a sequence defined by a test workflow to analyse the sample comprised in the sample container and to upload a result into the management system of an operator of the analytical system. Generally, each of the pre-analytical, analytical, post analytical, and transport means comprise an optical identifier reader capable of reading the visual identifier applied to the sample container. If the visual identifier is a barcode or a QR code, an optical barcode or QR code reader is used to verify each visual identifier of each sample container, for example. In another example, high-resolution cameras can be used to determine the identity of a code printed on the label associated with a sample container.

A variety of manufacturers often provide different elements of the pre-analytical, analytical, post analytical, and transport means, including optical means for reading the visual identifiers applied to the sample containers. In other words, different apparatuses comprised within the analytical system can comprise different optical identifier readers (for example, the optical identifier readers may be made by different manufacturers, have different optical arrangements, or be used in different illumination conditions). Therefore, different apparatuses have a differing ability to decode incorrectly applied, or damaged visual identifiers. There is a possibility that a damaged visual identifier could still be successfully decoded by a subset of the apparatuses comprised within the analytical system, but not all apparatuses. Furthermore, the ability of the same apparatus to decode visual identifier can vary over time as a sensor becomes affected by dust ingress, or if the apparatus experiences changes in illumination conditions, for example.

In other words, in a given set of analytical instruments of an analytical system, a subset of analytical instruments can read a specific visual identifier associated with a sample container, whereas another subset of analytical instruments cannot read the same visual identifier with the same probability of correct decoding. If an analytical instrument is unable to read a visual identifier, the sample is rejected, sorted out of the instrument (and/or transported to a holding area) and manual troubleshooting by lab staff as required. Such an approach is a manual burden for laboratories, but it is advantageous to prevent such errors as quickly as possible.

The principal problems associated with degradation of quality of visual identifiers are misalignment when placing an adhesively fixable visual identifier on a sample container, poor printing quality of a visual identifier owing to poorly maintained printer infrastructure at the sample collection site, and deterioration of the visual identifier during shipment.

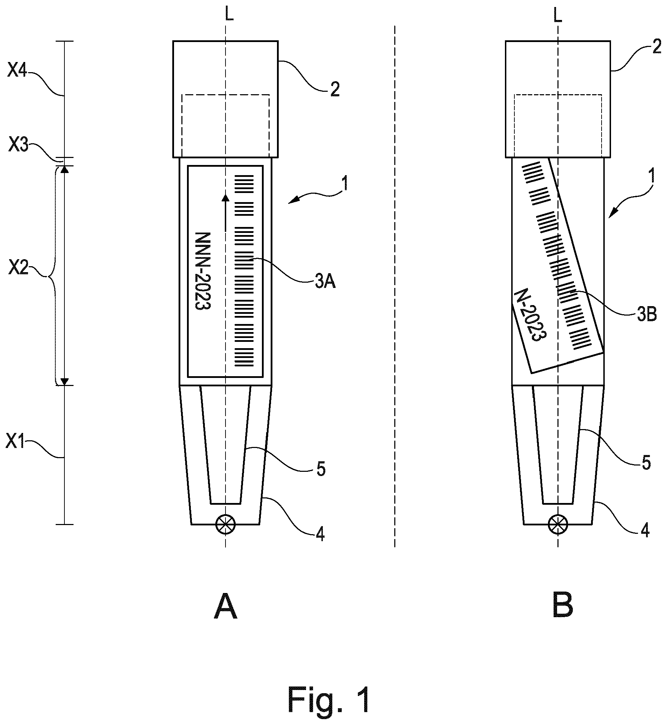

**FIG. 1** schematically illustrates two versions of a sample container having, respectively, a correctly applied visual identifier and an incorrectly applied visual identifier.

A sample container 1 in version A of **Fig. 1** comprises a container body 4 with a tapered proximal end. The illustrated sample container 1 is an integrated false bottom tube comprising a screw cap 2 and a tapering sample storage portion 5 at the proximal end. The sample container 1 is aligned along a longitudinal axis L. A visual identifier 3A is associated with the sample container 1. In the illustrated example, the visual identifier 3A is an adhesive label alphanumeric code and a barcode printed thereon.

Measured from an origin at the proximal end of the sample container 1, longitudinal dimension X1 defines the proximal extent of the visual identifier 3A on the body of the sample container 1. Longitudinal dimension X2 defines a longitudinal dimension of the visual identifier 3A. Longitudinal dimension X3 longitudinal separation between a distal extent of the visual identifier 3A on the body of the sample container 1 and the proximal extent of the cap 2. Longitudinal dimension X4 defines the longitudinal extent of the cap 2. Typically, the sample container 1 is a tube having a circular cross-section and thus an angular extent of the visual identifier 3A around the longitudinal axis L of the sample container 1 can also be defined.

During sample container preparation 1, a member of laboratory staff may position a printed visual identifier 3A on the side of the sample container one in accordance with the alignment illustrated in Fig. 1, case A, and in an example, this may be considered to be a correct placement enabling the visual identifier to be correctly decoded 3A by all apparatuses comprised in an analytical system.

Turning to the example of **Fig. 1****,** case B, the visual identifier 3B has been incorrectly applied by a member of lab personnel at an acute angle to the longitudinal axis L of the sample container 1. This results in a portion of the barcode contained on the visual identifier 3B being outside the field of view of a plurality of optical identifier readers used by analytical apparatuses of an analytical system. In other words, it is not possible to obtain the full barcode of visual identifier 3B because the angular placement of the visual identifier 3B on the external body of the sample container 1 means that there is no angular displacement of the sample container 1 around its longitudinal axis L at which the visual identifier 3B can be fully decoded. Therefore, many optical identifier readers or cameras would be unable to read visual identifier 3B.

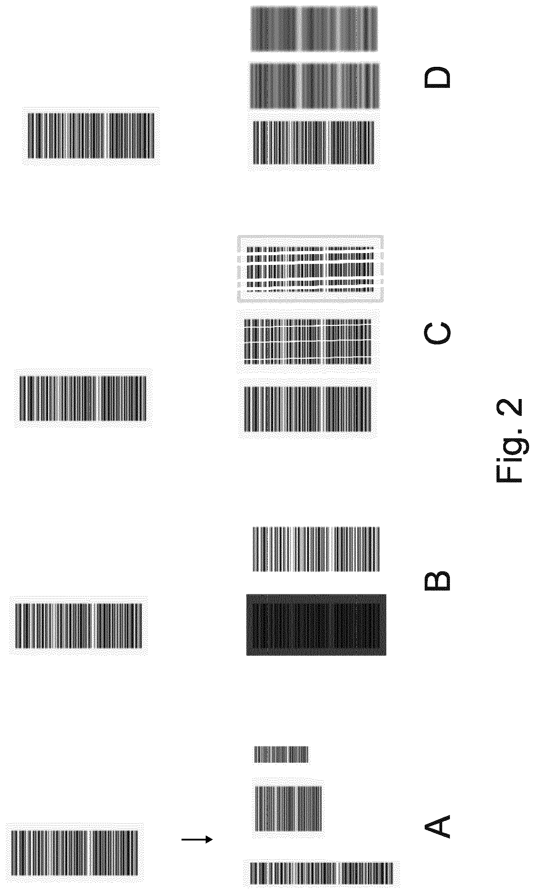

**FIG. 2** schematically illustrates four examples of barcode quality degradation.

The foregoing discussion concerned one variety of misplacement of visual identifiers associated with incorrect geometrical placement of the visual identifier 3B relative to the body of the sample container 1. Typically, such incorrect geometrical placement is attributable to laboratory staff error. **FIG. 2** provides examples of other problems that can affect visual identifiers.

**FIG. 2****,** case A illustrates the incorrect sizing of barcodes.

**FIG. 2****,** case B illustrates the provision of a barcode with different contrast or brightness ranges.

**FIG. 2****,** case C illustrates barcodes comprising longitudinal and/or lateral printing errors or scratches.

**FIG. 2****,** case D illustrates barcodes affected by blurriness, focus problems, or low print resolution.

According to an embodiment, the quality of a visual identifier can be assessed by a verifier. Verifiers are available for checking the quality of linear barcodes and QR codes, for example. A verifier can check the quality of linear barcode according to one, or more, of the ANSI X3.182, EN 1635, and ISO 15416 standards. The performance of optical identifier readers of analytical devices comprised within an analytical system is also rateable according to one, or both, of the ANSI X3.182 and EN 1635 standards.

Accordingly, one approach to ensuring, or improving, the reading ability of visual identifiers before they enter an analytical system is to use a verifier to check the quality of the visual identifiers according to one or both of the quality scales defined by the ANSI X3.182, EN 1635, and ISO 15416 standards. The rating obtained by the verifier is compared to the known reading performance of optical identifier readers comprised within apparatuses of the target analytical system.

A verifier according to the IS0 15416 standard broadly classifies linear barcodes into the following five categories:
Grade 0: the barcode is unreadable or unscannable. A complete failure in terms of print quality.
Grade 1: the barcode is partially readable but is generally considered of poor quality. It may not scan reliably and is not suitable for practical use.
Grade 2: the barcode is of fair quality. It is generally readable and scannable, but it may not perform optimally in all environments or with all types of scanners.
Grade 3: the barcode is of good quality. It is reliably readable and scannable under most normal conditions.
Grade 4: the barcode is of excellent quality. It is highly reliable and readable across a wide range of conditions and with various types of scanners.

ISO/IEC 18004:2015 is an international standard that defines the specifications for the encoding, structure, and quality of Quick Response (QR) codes. Published by the International Organization for Standardization (ISO) and the International Electrotechnical Commission (IEC), this standard provides a comprehensive set of guidelines for creating and using QR codes. The quality -related aspects of ISO/IEC 18004.2015 relate to:
Symbol Contrast - measuring the difference in light reflectance between the dark and light elements of the QR code. A higher contrast ratio ensures better readability.

Modulation: the size ratio between the smallest and largest module (square) in a QR code. The modulation ratio affects the clarity of the code.

Fixed Pattern Damage: whether, or not, distortions are present in the QR code's fixed patterns, which are the alignment patterns and timing patterns. Distortions in these patterns can lead to decoding errors.

Unused Error Correction Capacity: QR codes typically contain error correction information to help recover data if the code is partially damaged. Unused error correction capacity indicates a lower likelihood of decoding errors.

Print Growth: how much the QR code grows or shrinks compared to the original design. Proper print growth ensures accurate scanning.

Reflectance Margin: the acceptable range of reflectance values for the light and dark elements of the QR code.

Axial Non-uniformity: asymmetry of the QR symbol at symbol edges and corners.

Grid Non-uniformity: irregularities in the grid structure of the QR code.

Decode: whether, or not, when decoded, the QR code contains the correct data.

Compliance with ISO/IEC 18004:2015 ensures that QR codes are created and used consistently, allowing for reliable scanning and interpretation across different devices and environments. This standard plays a crucial role in the widespread adoption and effective use of QR codes in various industries and applications.

The ISO/IEC 18004:2015 quality rankings broadly classify QR code quality according to the forementioned aspects by:
Grade 0: QR code is unreadable or unscannable. It signifies a complete failure in terms of print quality.
Grade 1: QR code is partially readable but is generally considered of poor quality. It may not scan reliably and is not suitable for practical use.
Grade 2: QR code is generally readable and scannable, but it may not perform optimally in all environments or with all types of scanners.
Grade 3: good quality. QR code is reliably readable and scannable under most normal conditions.
Grade 4: excellent quality. QR code is highly reliable and readable across a wide range of conditions and with various types of scanners.

If all optical identifier readers comprised within analytical devices of the analytical system can read a visual identifier under test when that visual identifier is rated by a verifier, then a sample container associated with the visual identifier under test can be admitted to the analytical system with a high degree of confidence that all analytical apparatuses within the analytical system can read the visual identifiers, and thus no system stoppage requiring human intervention will occur.

Some visual identifier verifiers also check for geometrical placement abnormalities as illustrated, for example, in **Fig. 1****,** case B.

Furthermore, aspects of visual identifier verification may be performed by obtaining an image or a video of a visual identifier under test, and performing image or video analysis on the visual identifier depicted in the image or video. Image or video analysis of a visual identifier can be performed in combination to, or as an alternative, to using barcode or QR code verification hardware.

Accordingly, in an embodiment, the ability of an optical identifier reader to read a visual identifier associated with the sample container can be performed using a stand-alone visual identifier verifier, image or video-based techniques, or a combination.

One example of a stand-alone barcode verifier is the Omron (TM) Microscan (TM) LVS-9510, however a skilled person will appreciate that a wide variety of verifiers from other manufacturers can be used to assess the quality of a visual identifier.

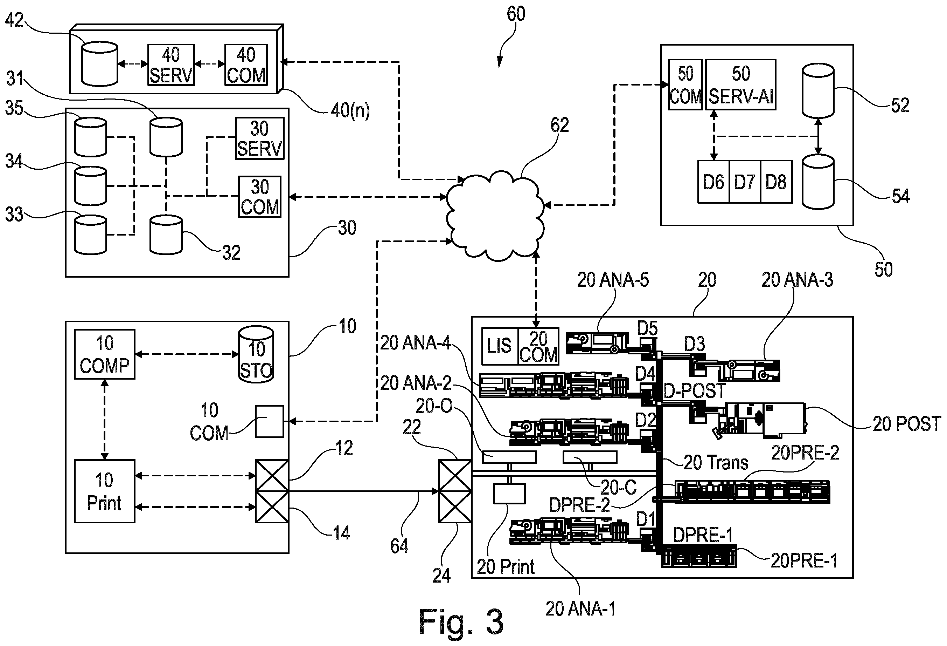

FIG. 3 schematically illustrates an example of a system for sample processing.

In embodiments, the system 60 is distributed over at least a sample collection facility 10 and an analytical system 20. In some embodiments, the system 60 comprises one, or more, of a data processing agent 30, one or more manufacturer data stores (40(n)), and an analyser monitoring agent 50.

The sample collection facility 10, or sample reception, is responsible for collecting biological samples from patients prior to analysis in the analytical system 20. The biological sample is obtained from the patient and isolated in a sample container 1 at the sample collection facility, for example. The sample container 1 is labelled with a visual identifier (visual indication) enabling an analytical system 20 to subsequently identify the sample container 1. In embodiments, the sample collection facility 10 is a phlebotomy department of a hospital, a remote doctor's surgery or outpatient clinic. In embodiments, the sample collection facility 10 is not located in a fixed building but may be considered to be provided by a health visitor able to visit patients and obtain patient samples at the patient's home.

According to one embodiment, the sample collection facility 10 may comprise a computer 10-COMP, a printer 10-PRINT configured to print visual identifiers for application to one or more sample containers 3A, an optional data storage element 10-STO, and a communication gateway communicably coupled to a Wide Area Network 62. The sample collection facility 10 may further comprise one, or both, of an optical identifier reader 12 and/or a camera 14 configured to obtain a digital representation of a visual identifier 3A associated with a sample container 1. The optical identifier reader 12 and/or the camera 14 may be operatively connected to the computer 10-COMP. The computer 10-COMP may host device driver software enabling a software environment of the computer 10-COMP to obtain a classification result characterising the sample container associated with the visual identifier from one or more of the optical identifier reader 12 and/or the camera 14.

According to a further embodiment, pre-labeled tubes are provided. In this case, the labels are not printed at the sample collection facility 10. Thus, the sample collection facility 10 may not comprise a printer 10-PRINT. Instead, the samples or tubes are pre-labeled with an identifier. The pre-labeled identifier is processed or checked as the printed visual identifiers.

The computer 10-COMP is also configured to host sample registration software enabling a link between a visual identifier associated with the sample container and a unique database identifier to be registered in a database hosted either at the sample registration location (10-STO) or in a remote database located in off-site data centre 30. The computer 10-COMP is also configured to host a device driver of printer 10-STO. For example, as part of the sample accession process, sample registration software operating on the computer 10-COMP assigns a unique database identifier to a registered sample container. The computer 10-COMP may, for example, instruct the printer 10-PRINT to print a unique label comprising a visual identifier, wherein the visual identifier is logically linked to the unique database identifier generated during the sample accession process by the computer 10-COMP.

A healthcare professional operating the computer 10-COMP and responsible for overseeing the sample accession process may, for example, attach the printed label to the sample container 1. In examples, the sample accession process may also comprise the healthcare professional selecting one, or more, test orders (order data 34) to be performed on the sample comprised in the sample container 1. The healthcare professional uses an interface provided by sample reception interface software on computer 10-COMP to select one, or more, test orders. The selected one, or more, test orders are logically linked to the unique database identifier associated with the sample container. The computer 10-COMP may, for example, update remote databases with the test orders assigned to each unique database identifier associated with the sample container.

Once a sample has been obtained from a patient, secured in a sample container 1, and registered to a unique database identifier that is logically linked to the visual identifier printed and secured to the sample container, the historical approach has been to send the one or more sample containers to an analytical system 20 (such as a central IVD laboratory) using, for example, a medical courier service 64.

Problematically, however, sample containers having defective or damaged visual identifier 3B can then enter the analytical system 20 without oversight. Some analytical apparatuses may not be able to read such defective or damaged visual identifiers 3B, implying that to improve efficiency, the visual identifier should be replaced before the relevant sample containers enter the analytical system 20. On the other hand, it may be possible to determine that a visual identifier are slightly damaged, but that the analyses nominated to satisfy a particular test order are still able to handle a visual identifier with slight defects. In this case, replacing the label would introduce inefficiency.

Therefore, the sample collection facility 10 comprises one or more of an optical identifier reader 12, and/or a camera 14. In an example, the camera 14 can be comprised in a smart phone, or smart tablet, for example. According to an example, the functionality of the computer 10-COMP, storage means 10-STO, and camera 14 are performed by a smart phone or smart tablet.

In an embodiment where the optical identifier reader 12 is a verifier, the classification result may comprise an assessment of a visual identifier according to one or more of the ANSI X3.182, EN 1635, and ISO 15416 standards, for example. In an embodiment where the optical identifier reader 12 is a camera, the classification result may comprise an assessment of a visual identifier according to one or more of the ANSI X3.182, EN 1635, and ISO 15416 standards, for example. In an embodiment where the optical identifier reader 12 is a camera, the classification result may comprise an assessment of the geometric placement of the visual identifier on a given sample container.

Prior to the healthcare professional admitting the one or more sample containers 3A to the medical courier service 64 for delivery to the analytical system 20, healthcare professional can examine the quality of the visual identifier of the sample container 1 using one or more of the optical identifier reader 12 and/or a camera 14, optionally in view of the test that the healthcare professional intends to order from the analytical system 20. For example, the healthcare professional examines the sample container 1 bearing the label printed by printer 10-PRINT using the optical identifier reader 12 (verifier) and/or the camera 14.

The computer 10-COMP compares the quality classification of the visual identifier with registered limitations of optical identifier readers or cameras comprised within the apparatuses of the analytical system 20. If the computer 10-COMP receives information that at least one visual identifier does not meet an intended quality classification, the computer 10-COMP alerts the healthcare professional to this fact so that the healthcare professional can correct the problem with the visual identifier before the sample container to which the visual identifier is attached is sent to the medical courier 64. For example, resolving a defective visual identifier may require applying a new label on top of a misaligned old label. In another example, resolving a defective visual identifier may require printing a new label with a different code, to avoid a temporarily defective pixel in the printer 10-PRINT. After printing the new label and attaching it to the sample container, the likelihood that the analytical system 20 is interrupted by a defective visual identifier is significantly reduced.

The analytical system 20 is, for example, an in vitro diagnostic (IVD) laboratory. Processing a sample container analytical system 20 can, for example, comprise admitting a plurality of sample containers comprising corresponding visual identifiers into the analytical system 20. The sample containers are, for example, IVD containers such as IVD tubes; the IVD containers may be held in IVD container holders such as IVD tube racks. The analytical system 20 is configured to perform pre-analytical workflow steps on the samples (e.g. preparatory steps such as centrifuging). The analytical system 20 is configured to perform analytical workflow steps on the samples (e.g. adding a reagent to the sample and measuring the reaction of the sample with the reagent). The analytical system is configured to perform post-analytical steps on the samples (e.g. storage of a sample in a refrigerator for later use).

Apparatuses comprised in the analytical system 20 are typically categorized according to the different type of sample processing steps they can perform. A transport IVD laboratory instrument 20Trans is designed for transporting samples (resp. the sample containers and/or respective holders), e.g. from one analytical apparatus to another. A pre-analytical IVD laboratory instruments 20PRE-1, 20PRE-2 is designed for performing pre-analytical steps on the samples. An analytical IVD laboratory instrument 20ANA-N is designed for performing analytical steps (such as an analytical test) on the samples; an analytical IVD laboratory instrument 20ANA-N can comprise a digital analytical IVD laboratory instrument designed for performing analytical computation steps (e.g. a medical algorithm). A post-analytical IVD laboratory instrument 20POST is designed for performing post-analytical steps on the samples, and/or sample storage. Some analytical apparatuses 20 are capable of performing multiple type of sample processing steps, e.g. pre-analytical and analytical steps.

The analytical system 20 therefore comprises one or more analytical apparatuses 20ANA 1-4 designed for processing samples, e.g. for performing one or more steps of an intended workflow on the sample. Processing a sample can comprise one or more physical processing steps (e.g. moving, mixing, heating, etc.). An analytical apparatus 20ANA 1-4 can comprise instrument hardware for processing samples (e.g. gripper, reagent storage, pipetting apparatus, heating element, etc.) as well as instrument software designed for operating the instrument hardware. An analytical apparatus 20ANA 1-4 can comprise a control unit designed for controlling, in particular steering, the operation of the instrument hardware, wherein the instrument software can be designed for being executed using a control unit.

According to an embodiment, each analytical apparatus ANA 1-4 comprises at least one optical identifier reader or camera D1-D4. In an example, the reading capabilities of the optical identifier readers or cameras D1-D4 are characterized by the manufacturers of the optical identifier readers according to one or more of the ANSI X3.182, EN 1635, and ISO 15416 standards, or comparable standards for QR code readers.

A first analytical apparatus ANA 1 may comprise an optical identifier reader having a different capability for reading visual identifier (for example, linear barcodes and/or QR codes) compared to other analytical apparatuses ANA 2-4 in the analytical system 20.

In an example, the reading capabilities of the optical identifier readers D1-D4 are characterized according to a measurement or calibration measurement carried out in-situ in the analytical system 20.

In an example, the reading capabilities of the optical identifier readers D1-D4 are characterized according to a drift model or machine learning model characterising the change in decoding capability of a respective type of optical identifier reader or camera over time.

The analytical system 20 may further comprise a laboratory information system (LIS) that is communicably coupled to the other equipment in the analytical system 20. The LIS, for example, interfaces with, or hosts, middleware of the analytical system 20 configured to operate the apparatuses 20ANA-N, 20PRE-N, 20POST-N and transport system 20Trans of the IVD laboratory based on service requests or workflows generated by the LIS. According to an example, the service requests or workflows are generated by the LIS based on order data 34 generated from a test order selected by a healthcare professional at a sample reception facility 10 and logically linked to a particular sample container 1.

The LIS may collect and store, or forward sample analysis results apparatuses 20ANA-N as they are generated. The LIS may monitor reagent or consumable usage in the analytical system 20, and generate requests for new orders of reagents or consumables when current stocks of reagents or consumables are falling low. The LIS may collect information concerning maintenance needs or analyser non-compliances and generate requests for engineering checks.

According to an embodiment, the LIS is configured to coordinate calibration, or to obtain capability definitions of optical identifier readers (and/or cameras) D1-D5, DPRE, DPOST associated with apparatuses of the analytical system 20. Furthermore, the LIS may gather information and generate statistics defining the performance of the overall IVD laboratory. The LIS may host an information source, such as an embedded website, enabling laboratory staff and engineering staff to assess the performance of the IVD laboratory, or any apparatus within it.

The analytical system 20 may further comprise a communications interface 20COM enabling the LIS and/or the apparatuses 20ANA-N, 20PRE-N, 20POST-N and transport system 20Trans of the analytical system 20 to be communicably coupled to one, or more, of the sample reception facility 10, the data centre 30, the set of manufacturer data stores 40(n), and/or the analyser monitoring agent 50 via the Wide Area Network 62.

The sample entry point of the analytical system 20 optionally comprises one, or both, of an optical identifier reader 22 and a camera 24. This enables verification of visual identifiers of sample containers at the entry point to the analytical system 20. For example, a sample reception facility 10 may not be equipped with an optical identifier reader 12 and/or an appropriate camera 14 enabling a classification result characterising the visual identifier to be obtained. In this case, the visual identifier of sample containers can be examined at the entry point to the analytical system 20. Instead, or in addition, if a non-compliant sample container is detected by optical identifier reader 22 and/or camera 24, a staff member of the facility hosting the analytical system 20 can redirect the non-compliant sample container 1 for relabelling before it enters the analytical system 20. This approach also enables the detection of damage to visual identifiers during the medical courier process. If a sample container comprising a visual identifier fails an admission check to the analytical system 20 carried out by optical identifier reader 22 at the analytical system 20, but the same sample container previously passed a checkout test performed by an optical identifier reader 12 located at the sample reception facility 10, a useful additional conclusion can be made that the visual identifier 3A has been damaged during sample transport.

The analytical system 20 can, in an embodiment, further comprise an internal visual identifier quality monitor 20-O. The visual identifier quality monitor 20-0 can comprise one or more of an optical identifier reader such as a barcode reader, and/or a camera. The purpose of the visual identifier quality monitor 20-0 is to withdraw one or more sample containers from the transport system 20Trans of the analytical system 20 and to test the quality of the visual identifier of one or more of the sample containers. This enables the analytical system 20 to periodically ensure that visual identifier degradation is not occurring within the analytical system 20 itself. Furthermore, if a sample container 1 comprising a sufficiently degraded visual identifier is discovered, the analytical system 20 can dispatch that sample container to an automated sample printer 20Print, so that a visual identifier of improved quality can be applied automatically to the sample container (for example, over the surface of the degraded visual identifier).

In an embodiment, if one or more optical identifier readers and/or cameras of the analyser system 20 fails to read a visual identifier associated with a sample container to a satisfactory level, the laboratory information system LIS can detect this and redirect the faulty sample container to the internal visual identifier quality monitor 20-0. The internal visual identifier quality monitor 20-0 obtains an image or analysis of the faulty sample container, and its visual identifier. In this way, examples of problematic visual identifiers from a real laboratory context can be collected and used for the training of a machine learning model, for example.

According to an embodiment, the analytical system 20 further comprises a calibration standard store 20-C. The calibration standard store 20-C comprises a number of placebo sample containers comprising visual identifiers having a range of conditions according to one of the relevant industry standards defined previously, and/or having a range of simulated fixation abnormalities and scratches. In an embodiment, the placebo sample containers may be provided by manufacturers of one or more of the apparatuses of the analytical system 20.

The purpose of the calibration standard store is to utilize periods of low utilization of the analytical system 20 to calibrate optical identifier readers D1-D5 within the analytical system, or at least to monitor degradation of the readers. For example, a sequence of placebo sample containers having gradually declining readability can be repeatedly directed to analyser 20ANA-3. The laboratory information system LIS collects information from a control unit of analyser 20ANA-3 about whether, or not, each placebo sample container can be correctly read and decoded. When the calibration run is complete, the laboratory information system LIS can send the results to, for example, a manufacturer database 40(n) to enable the scheduling of a service. Alternatively, the LIS can send the results to a data processing agent 30SERV, so that if the optical identifier reader of a first analyser begins to behave erratically, the data processing agent adjusts test orders (order data 34) requiring the first analyser to a substitute analyser. Furthermore, the LIS can send the results of the calibration run to an analyser monitoring agent 50.

The system 60 further comprises a data centre 30 that is communicably coupled to at least the analyser system 20 and the sample reception facility 10 via the Wide Area Network 62.

The data centre 30 comprises a communication gateway 30 that is communicably coupled to a data processing agent 30SERV.

The term "data processing agent" refers to a computer implemented software module executing on one or more computing devices, such as a server, which is able to receive analytical device status data from a laboratory information system LIS of the analytical system 20, and sample accession information from a computer 10COMP of a sample reception facility 10, for example. The data processing agent 30SERV may be implemented on a single server, or multiple servers, and/or an internet-based "cloud" processing service such as Amazon AWS (TM) or Microsoft Azure (TM). The "data processing agent", or a portion of it, may be hosted on a virtual machine. The data processing agent can receive, process, and transmit operational information and data to the sample reception facility 10, the analytical system 20, manufacturer data service 40(n), and an analyser monitoring agent 50.

For convenience, the data centre 30 is illustrated as hosting the data processing agent 30SERV, although a skilled reader will appreciate that the data processing agent 30SERV may be hosted at a variety of locations, such as within the analytical system 20, or even at the sample reception facility 10. Furthermore, the data processing agent 30SERV is, in embodiments, configured to host web applications accessible by smart phone, tablet, or computers for conducting a sample accession process, or a lab management process, or to obtain test results.

The data centre comprises a first data store 31 comprising laboratory configuration information. The laboratory configuration information comprises records defining the system architecture of one or more analytical systems 20, for each one or more analytical systems 20 available to a healthcare professional ordering tests from the sample reception facility. For example, the laboratory information specifies the type number of each apparatus present in the analytical system 20, and the interconnections between the apparatuses.

The data centre comprises a second data store 32 comprising visual identifiercompatibility information of a range of analytical apparatuses. Accordingly, for each apparatus 20ANA-1 comprised in a given analytical system 20, the rules governing the capability definition of an optical identifier reader or camera D1 of that apparatus 20ANA-1 are stored in the visual identifier compatibility information. The visual identifier compatibility information can be a set of generic rules defined by a manufacturer of an analytical device. In some cases, the visual identifier compatibility information can be updated based on calibration runs performed, for example, using the calibration standards of the calibration standard store 20-C. In some cases, the visual identifier compatibility information can be updated based on a download or an update from one or more manufacturer data stores 40(n).

The data centre comprises a third data store 33 comprising calibration information, for one or more of the apparatuses 20ANA-1. Calibration of optical identifier readers D1-D5 can be performed manually by laboratory staff, or automatically using calibration runs coordinated from the calibration standard store 20-C. The degree of drift of the capability of opticalidentifier readers D1-D5 to successfully read calibration standards over time is, thus, registered in the third data store and can be used to more accurately predict, for a specific analyser system, whether a visual identifier attached to a sample container will be read successfully, or not, based on a measured state of the optical indication of the analyser system.

The data centre comprises a fourth data store 34 comprising order data. Order data comprises a first identifier of at least one type of analytical test to be performed by an analytical system 20 using the sample container 1, and/or at least one transfer action of the sample container 1 within the analytical system 20. The order data optionally comprises a second identifier for identifying a specific analytical system 20 to be used to perform the at least one analytical test. In other words, the order data defines workflow steps and specific types of analyser that a sample container must be processed by in order to obtain a test result.

The data centre comprises a fifth data store 35 comprising sample accession data. This database comprises patient-specific information such as the unique sample container identifier of a sample, one or more test types that have been ordered in respect of the sample, linked to a (typically anonymized) code that can be used to identify the patient.

The fifth data store 35 may also comprise a link or database key to a results database (not illustrated). When a new test is ordered at a sample reception facility, a new entry comprising sample accession data is entered into the fifth data store 35 comprising a unique sample container identifier that is used to generate a visual identifier of a sample container.

The manufacturer data stores (40(n)) are accessible via gateway 40COM. For each type of apparatus 20ANA-1-5, 20PRE-1,2, 20POST, and 20Trans in the analytical system 20, the relevant manufacturer can provide an apparatus specification comprising a capability definition of an optical identifier reader and/or camera of the apparatus. An application programming interface (API) 40SERV can enable access to a data store 42 comprising a range of capability definitions for different types of manufacturer equipment, for example.

In an embodiment, the data processing agent 30SERV is configured to populate the second data store 32 with visual identifier compatibility information downloaded from at least one manufacturer data store 40(n). In another embodiment, direct connection to a manufacturer data store 40(n) is not required, and the visual identifier compatibility information can be provided to the second data store by manual entry, for example.

The analyser monitoring agent 50 can be hosted by a remote server or cloud service, or can be comprised within the data centre 30 and/or analyser system 20. The purpose of the analyser monitoring agent 50 is to monitor and predict changes to types of visual identifier readers and/or cameras utilized by apparatuses used in the analyser system 20. Therefore, the analyser monitoring agent 50 can comprise a database 52 of visual identifier reader definitions, optionally obtained from one or more manufacturing data stores 40(n). The analyser monitoring agent 50 can comprise a database 54 of successfully and unsuccessfully decoded visual identifiers obtained by the internal visual identifier quality monitor 20-O. The analyser monitoring agent can comprise one, or more, digital models D6, D7, D8 of visual identifier readers. Optionally, the digital models are based on machine learning models trained on the information comprised in the database 54 of successfully and unsuccessfully decoded visual identifiers obtained by the internal visual identifier quality monitor 20-O.

The elements of the system 60 mentioned previously are typically geographically distributed. Data communication links between elements of the system 60 are provided, for example, by a Wide Area Network (WAN).

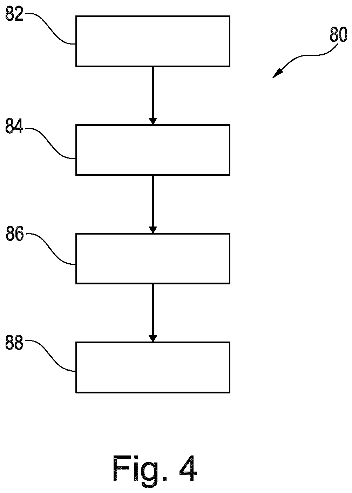

**FIG. 4** schematically illustrates a computer implemented method according to the first aspect.

According to the first aspect, there is provided a computer implemented method 80 for analytical sample container classification, wherein the method comprises:
- obtaining 82 a digital representation of a visual identifier 3A associated with a sample container 1;
- identifying 84 at least one apparatus 20PRE-1 comprised within an analytical system 20, wherein the analytical system 20 is intended to perform at least one analytical test using the sample container 1, wherein the at least one apparatus 20PRE-1 comprises an optical identifier reader or camera;
- classifying 86 the sample container 1 associated with the visual identifier 3A by characterizing the ability of the optical identifier reader or camera of the at least one apparatus 20PRE-1 comprised in the analytical system 20 to decode the visual identifier 3A associated with the sample container 1, to thereby generate a corresponding classification result characterizing the sample container 1 associated with the visual identifier 3A; and
- outputting 88 a message defining the classification result.

According to an example, the digital representation of visual identifier 3A is a digital representation of a barcode, QR code, or plain text comprised on the visual identifier 3A. The digital representation of the visual identifier 3A may comprise a digital image, but also parameters defining a visual identifier such as a barcode or QR code as defined in the ANSI X3.182, EN 1635, and ISO 15416 standards, for example. The digital representation of the visual identifier 3A can comprise analysis results obtained from an optical verifier for verifying the quality of a barcode and/or a QR code.

According to an embodiment, after obtaining the digital representation of a visual identifier 3A associated with a sample container 1, the digital representation of a visual identifier 3A is processed using an image processing algorithm to thus determine one or more attributes defining the quality of the visual identifier.

According to an embodiment, obtaining the digital representation of a visual identifier 3A associated with a sample container 1, comprises reading the visual identifier 3A with a bar code or QR code reader, to thus determine one or more attributes defining the quality of the visual identifier.

According to an embodiment, the one or more attributes of the visual identifier comprise measures of a range of acceptable alignment deviations of the visual identifier relative to a longitudinal axis of a sample container; a range of acceptable occlusions of the visual identifier; a range of acceptable vertical and/or horizontal dimensions of the visual identifier; a range of acceptable blurring or resolution artefacts of the visual identifier; a range of acceptable contrast or brightness ratios of the visual identifier; and/or a range of acceptable artefacts of the visual identifier; and /or the one or more attributes of the visual identifier comprise at least one, or any combination, of a barcode edge determination metric, a barcode minimum reflectance metric, a barcode minimum edge contrast metric, a barcode symbol contrast metric, a barcode modulation grading, a barcode defects grading, and/or a barcode reading grading.

For example, the digital representation of visual identifier 3A and/or capability definition 32 of the optical identifier reader of the at least one apparatus can characterize at least one, or any combination, of a range of acceptable alignment deviations of the visual identifier relative to a longitudinal axis of a sample container; a range of acceptable occlusions of the visual identifier; a range of acceptable vertical and/or horizontal dimensions of the visual identifier; a range of acceptable blurring or resolution artefacts of the visual identifier; a range of acceptable contrast or brightness ratios of the visual identifier; and/or a range of acceptable artefacts of the visual identifier.

According to an embodiment, the digital representation of visual identifier 3A and/or capability definition 32 of the optical identifier reader of the at least one apparatus further characterizes at least one, or any combination, of a barcode edge determination metric, a barcode minimum reflectance metric, a barcode minimum edge contrast metric, a barcode symbol contrast metric, a barcode modulation grading, a barcode defects grading, and/or a barcode decodability grading.

The digital representation of the visual identifier can be a digital image of a visual identifier, or can be one or more metrics capable of characterising the assessment of a verifier of one or more of the aforementioned parameters. On the other hand, the capability definition 32 defines the ability of a given type of optical identifier reader to read a visual identifier rated by verifier to a given performance level.

Accordingly, available data sources are combined to form a prediction of sample visual identifier compatibility or validity for a specific customer lab instrument configuration, given a visual identifier of measured quality.

In one example, the proposed data combination comprises the following information set: laboratory configuration information (for example, stored in the first datastore 31) characterises, for a specific analytical system 20, the identities of apparatusesavailable in the analytical system 20. This data is stored in a laboratory specific configuration file 31 in a backend data storage solution in one example.

A second datastore 32 information about visual identifier compatibility of each analyser apparatus installed in the analyser system 20. In other words, what type of optical identifier reader is implemented in each apparatus present in the analyser system 20. According to an example, this can be determined by a factory test at a custom installation using a set of samples with varying barcode quality. Alternatively, this information can be obtained from a manufacturer database 40(n).

At the sample reception 10, actual visual identifier quality is obtained by a user front end device such as an optical identifier reader, and/or a camera located in a user mobile phone, or smart tablet, for example. In the case of a barcode, for example, the visual identifier quality is based on industry standard measures of reflectance, contrast, readability, and the like.

A further data set that can be used when performing the assessment of visual identifiercompatibility with an analyser system 20 is to combine the order data 34 associated with a sample container comprising a visual identifier. An analyser system 20 may comprise a large number of analysers, many of which may not be used for specific test orders (test orders are automatically broken down within the analyser system 20 into workflows by the laboratory information system). Therefore, a sample container with a visual identifier of average quality could still be admitted to the analytical system 20 provided a subset of analysers within the analytical system 20 can be identified that would work with a given visual identifier quality.

For example, the sample specific test orders are used to look up the corresponding apparatuses in the analytical system 20 required to analyse a specific test order. An apparatus specific visual identifier quality requirement is matched to the measured quality of the visual identifier to thus provide a classification result characterising the ability of each analyser required for a given test order to decode the visual identifier If all apparatuses of an analytical system 20 required to run a specific test order are determined to be capable of decoding specific visual identifier, the classification result is positive. If at least one apparatus of an analytical system 20 required to run a specific test order is determined to be incapable of decoding a specific visual identifier, the classification result is negative.

The added value of indicating sample barcode quality is that, at sample reception 10, it is possible to rule in or rule out a given sample container at lab sample reception 10, and/or reroute samples associated with a defective sample container dependent on the quality of the visual identifier associated with the sample container. This optimises troubleshooting of insufficient barcode label quality and can reduce the need for relabelling within the analytical system 20. The compatibility at the sample reception 10 can be performed using, for example, a mobile device or scanner comprising a camera (or a mobile device communicably coupled to a barcode or QR code verifier, for example). This provides prompt feedback to users at the sample reception 10 about the capability of a downstream analyser system intended to execute test order to handle the specific sample container comprising the visual identifier. Furthermore, the data collected can be used by a laboratory operator to analyse the root cause of insufficient visual identifier quality (for example, per collection site, to identify defective visual identifier printers, or to identify a need for staff training).

According to an embodiment, the method further comprises outputting a corresponding identifier of one or more sample containers comprising a visual identifier 3A that will receive a negative classification.

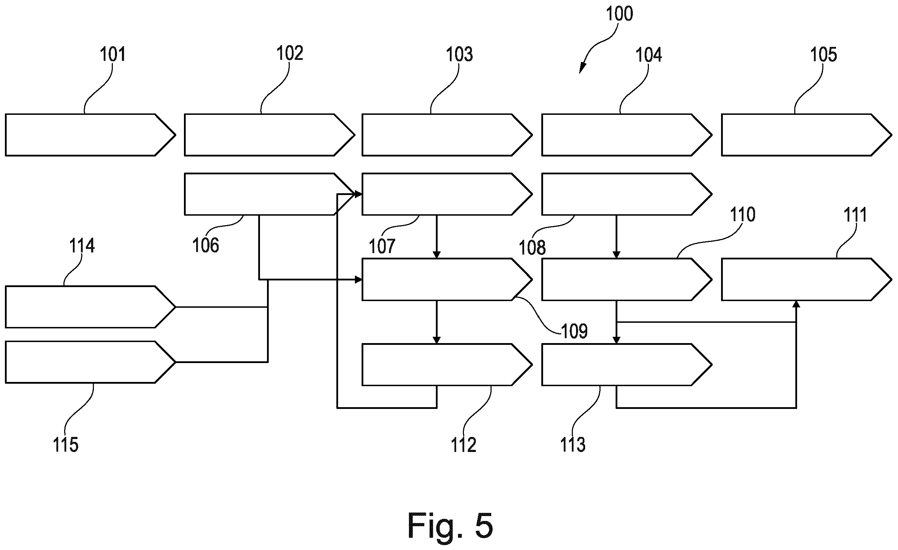

**FIG. 5** schematically illustrates an example 100 of a process for sample container examination.

When an analytical system 20 is configured or reconfigured at step 101, a laboratory instrument configuration file 114 is produced by a designer of the analytical system 20 which is provided to the data centre 30. The laboratory instrument configuration file 114 defines, for example, the interconnections between laboratory equipment, manufacturer identification information, and the like.

For one or more apparatuses comprised in the analytical system 20, instrument visual identifier quality parameters are obtained, for example from a manufacturer database 40(n) at step 115.

Column 102 of the process chart defines obtaining a test order, wherein a healthcare professional at a sample reception location 10 obtains a sample from a patient, and attaches a visual identifier to a sample container. A computer 10COMP at the sample reception location 10 is used to select a test order (order data 34) intended to be performed on the sample.

Before the sample container leaves the sample reception 10, a sampling step 103 comprises using an visual identifier reader 12 and/or camera 14 at the sample reception location 10 to perform a scan to measure the quality of the visual identifier 107. Once the quality of the visual identifier has been obtained, a check of barcode quality is performed at step 109 by comparing the quality of the visual identifier to the instrument visual identifier quality parameters for each analyser apparatus specified by the test order obtained in step 106. If the quality of the visual identifier is such that all analysers in the analyser system 20 required to perform a specific test order are capable of reading the visual identifier obtained at step 107, the sample container associated with the successfully checked visual identifier can be sent to the analyser system 20. Otherwise, at step 112 sample reception facility 10 is alerted to the problem of insufficient visual identifier quality. According to one option, the visual identifier is reprinted, enabling the healthcare professional to affix the new sample container and to rescan the sample container to verify that the visual identifier attached to the sample container has a satisfactory quality. According to another option, the workflow can be recompiled to find other analysers capable of reading the visual identifier and performing the relevant workflow step.

Column 104 of **Fig. 5** illustrates a set of the process flow that can be carried out alternatively, or in addition, to the process flow of 103. When the sample container arrives at the reception point of analyser system 20, the visual identifier quality is scanned at step 108 using an optical identifier reader and/or camera. A laboratory information system LIS (for example) of the analyser system 20 interrogates the lab instrument configuration file and the instrument barcode quality lookup information to determine whether, or not, the sample container comprising the scanned visual identifier can proceed into the analyser system 20, or whether the visual identifier should be replaced prior to the sample container proceeding into the analyser system 20. Therefore, there is an optional step 113 of providing a new visual identifier on the sample container at the collection site. When the sample container has a visual identifier of acceptable quality in view of the configuration of the analyser system 20 and the instrument barcode quality capabilities of analysers comprised within the analyser system 20, the sample container can be permitted to enter the analyser system 20.

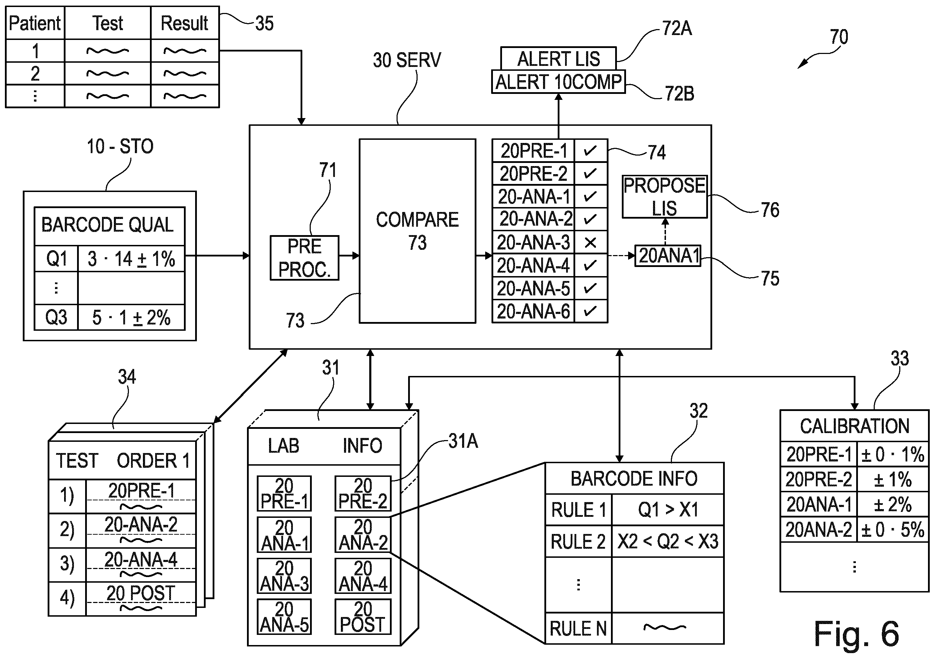

**Fig. 6** schematically illustrates a data model 70 for sample container examination.

In an exemplary setup, a specific test order and a lab analyzer config table are provided which define the used analyzer e.g., in form of a matrix. The combination of the used analyzer and a specific barcode reader capability info of the used analyzer define the barcode quality need e.g., in form of a matrix. The barcode quality need plus the specific barcode quality measured by the device used at the collection site define an OK or nOK for the lab processing. Accordingly, an error message or an ok is sent to the operator.

Data processing agent 30SERV obtains a digital representation of a visual identifier from, for example, a data storage device 10-STO located at a sample reception facility 10. In an embodiment, the digital representation of the visual identifier is associated with quality metrics based on industry standards of the visual identifier obtained, for example, using an optical identifier reader and/or a camera at the sample reception facility 10. In other words, the digital representation of the visual identifier characterises the quality of visual identifier under test at the sample reception facility 10. In variations, the digital representation of the visual identifier may be obtained at the sample entry point to an analytical system 20, or from within the analytical system 20.

Data processing agent 30SERV is configured to obtain laboratory configuration information from, for example, a first data store 31. The laboratory configuration information contains records 31A representing each functional apparatus comprised in an analytical system 20.

Data processing agent 30SERV can obtain information about a visual identifier compatibility of each apparatus comprised in the analyser system 20. For example, the visual identifier compatibility can be determined by one or more rules that a visual identifier 3A of a sample container should obey in order for the associated apparatus to be capable of reading a corresponding visual identifier. The rules can define, for example, geometric quantities such as a limit to the angular offset of a visual identifier relative to the longitudinal axis of the sample container. The rules can define, for example, maximum and minimum reflectance values as measured by a barcode verifier. Further visual identifier quality metrics are discussed elsewhere in the specification and all, or any combination, of such quality metrics can be used as a rule set.

Data processing agent 30SER optionally obtains, for at least one item of apparatus in the analyser system 20, calibration information from the third data store 33. The calibration information 33 can, for example, characterise how far the optical detection characteristics of a generic analyser comprised in the laboratory information of the first datastore 31 has drifted from its generic definition.

Data processing agent 30SERV can obtain a test order 34 relating to a test to be performed on a specific sample container identifiable by visual identifier.

Data processing agent 30SERV can obtain, from a fifth datastore 35, sample accession data. A sample accession database comprises patient-specific information such as a unique sample container identifier of a sample, one or more test types that have been ordered in respect of sample, linked to an anonymized code that can be used to identify the patient.

In use, a pre-processor 71 identifies that a new patient test has been ordered for a specific analyser system 20. The specific test that has been ordered is looked up in the set of test orders 34 (order data). The pre-processor 71 defines the laboratory characterising the specific analyser system 20 from database 31. The preprocessor 71 obtains order data 34 of a test that a healthcare professional intends to order for a sample, where the order data is obtained from the database 34. The preprocessor 71 compiles a workflow using the order data 34 and the laboratory configuration information in the first datastore 31. The workflow defines, for example, the movements between pre-analytical or preprocessing, analytical, and post-analytical or postprocessing apparatuses in the analytical system 20.

The compilation performed by the preprocessor 71 results in a list of optical identifier readers that will be used to identify a visual identifier associated with a sample container, as the sample container is processed in the analytical system 20 according to the workflow.

A comparator module 73 obtains, for each optical identifier reader that will be used to identify visual identifier according to the workflow, and associated rule set from datastore 32. According to example, the rule set from datastore 32 can be supplemented with calibration information of each optical identifier reader of each relevant apparatus comprised in the specific analyser system 20 requested by the sample reception facility 10 personnel.

The comparator module 73 compares the representation of the visual identifier measured at the sample reception facility 10 with the relevant rules defined by the workflow in the associated rule set from datastore 32. The example of Fig. 6 illustrates a case where seven out of eight analysers can successfully decode the visual identifier having the characteristics measured at the sample reception facility 10. However, the analyser 20ANA-3 is not able to successfully decode the visual identifier having the characteristics measured at the sample reception facility 10. In the case of an unsuccessful comparison, a first alert message 72A is sent to the laboratory information system LIS of the analytical system 20. A second alert message 72B is sent to the computer 10COMP of the sample reception facility 10. User interface software hosted by the computer 10COMP of the sample reception facility 10 may, thus, prompt a user to address barcode readability problems that would prevent the sample container bearing the visual identifier that triggered the unsuccessful comparison to the analyser 20ANA-3.

In an embodiment, the data processing agent 30SERV can search the first database 31 for a substitute analytical apparatus capable of performing the test step that the analyser 20ANA-3 is not able to complete owing to an inability to read the associated with the sample container previously identified. In the illustrated case, the data processing agent 30SERV proposes the analyser 20ANA-1 as a substitute for the analyser 20ANA-3. Accordingly, the laboratory information system LIS is informed at step 76 that the analyser 20ANA-1 should substitute for the analyser 20ANA-3 at this workflow step.

According to an embodiment, the computer implemented method further comprises:
- obtaining a capability definition 32 of the optical identifier reader of the at least one apparatus 20PRE-1 comprised in the analytical system 20, wherein the capability definition 32 characterizes at least one aspect of the optical identifier reader of the at least one apparatus 20PRE-1 to at least one visual identifier 3A; and
- classifying the visual identifier 3A comprises comparing the capability definition 32 of the optical identifier reader of the at least one apparatus 20PRE-1 to the digital representation of the visual identifier 3A associated with the sample container 1.

For example, if the visual identifier is a barcode, the capability definition 32 may define that the optical identifier reader of at least one apparatus comprised in the analytical system is capable of readingbarcodes to one of a plurality of grades of an industry standard, for example to grade 0, grade 1, grade 2, grade 3, or grade 4 of the IS0 15416 standard.

For example, if the visual identifier is a QR code, the capability definition 32 may define that the optical identifier reader of at least one apparatus comprised in the analytical system is capable of reading QR codes to one of a plurality of grades of an industry standard, for example to grade 0, grade 1, grade 2, grade 3, or grade 4 of the ISO/IEC 18004:2015 standard.

A skilled person will appreciate that the capability definition 32 for each optical identifier reader of an apparatus does not need to be defined according to the aforementioned standards, and that a wide range of parameters accessible to, or measured by, an optical identifier reader such as a barcode verifier or QR code verifier can be used to characterise the capability definition 32.

According to an embodiment, identifying the at least one apparatus 20PRE-1 comprised within the analytical system 20 further comprises:
- obtaining order data comprising a first identifier of at least one analytical test to be performed by an analytical system 20 using the sample container 1, and/or at least one transfer action of the sample container 1 within the analytical system 20, wherein the order data optionally comprises a second identifier for identifying a specific analytical system 20 to be used to perform the at least one analytical test.

An analytical test ordered by a healthcare professional at the sample reception facility 10 may be decomposed into workflows in a preprocessing step 71 performed by a data processing agent 30SERV prior to a test being performed by an analytical system 20. Different analytical tests require a sample container to be processed according to different protocols by different analytical apparatuses. According to a first option, visual identifier compatibility can be performed with reference to a generic analyzer system based only on manufacturer information about the capability of the optical identifier readers D1-D5. Associating an identifier of a specific analytical system 20 with a test order enables calibration information of optical identifier readers associated with specific analyzer apparatuses to be incorporated into the determination of whether, or not, a sample container comprising a measured visual identifier can be accepted into an analytical system 20.

According to an embodiment, the method further comprises, if the sample container 1, and/or its associated visual identifier 3A are classified with a negative classification result of the at least one apparatus 20PRE-1, outputting a message defining that the visual identifier 3A associated with the sample container 1 has received a negative classification in respect of the at least one apparatus 20PRE-1.

According to an embodiment, if a sample container 1 receives a negative classification the computer at the sample reception facility 10 may be prevented from allowing the user to send the sample container 1 to the analytical system 20. According to an embodiment, the user may receive, via a graphical user interface of the computer at the sample reception facility 10, a message providing information on the defective parameters of the visual identifier, to simplify troubleshooting. According to an embodiment, if a sample container 1 receives a negative classification, the computer at the sample reception facility 10 may allow the user to send the sample container 1 to the analytical system 20, and simultaneously send a message to the laboratory information system of the analytical system 20 advising a staff member at the facility hosting the analytical system 20 of the need to relabel the sample container 1 before its admission into the analytical system 20.

According to an embodiment, the method further comprises, if the sample container 1, and/or its associated visual identifier 3A are classified with a negative classification result of the at least one apparatus 20PRE-1, outputting, to a laboratory information system associated with the at least one analytical system 20, a message comprising an identification code of a sample container 1 comprising the visual identifier 3A that has received a negative classification in respect of the at least one apparatus 20PRE-1 comprised in the at least one analytical system 20.

According to an embodiment, if a message indicating a negative classification is output, the system is configured to prevent the sample container from progressing to the at least one analytical system.

According to an embodiment, if a message indicating a negative classification is output, the sample container is sent to a relabeling device.

According to an embodiment, wherein the at least one analytical system 20 comprises a set of apparatuses that will be used to perform the at least one analytical test in a predefined sequence defined by the analytical test identified by the first identifier of the order data 34; the method further comprises:
- comparing a plurality of capability definitions 32 respectively corresponding to each apparatus 20PRE-1 of the set of apparatuses defined by the order data 34 with the digital representation of the visual identifier 3A; and
if the visual identifier 3A associated with the sample container 1 does not meet the capability definition 32 of at least one apparatus 20PRE-1 comprised in the set of apparatuses:
- outputting a message defining that the visual identifier 3A associated with the sample container 1 has received a negative classification in respect of the at least one apparatus 20PRE-1 comprised in the set of apparatuses, wherein the message optionally identifies each apparatus 20PRE-1 comprised in the set of apparatuses that triggered the negative classification.

According to an embodiment, wherein the at least one analytical system 20 identified by the second identifier comprises a set of apparatuses that will be used to perform the at least one analytical test in a predefined sequence defined by the analytical test identified by the first identifier; and further comprising:
- comparing a plurality of capability definitions 32 respectively corresponding to each apparatus 20PRE-1 of the set of apparatuses with the digital representation of the visual identifier 3A; and
if the visual identifier 3A associated with the sample container 1 does not meet the optical capability definition 32 of at least one apparatus 20PRE-1 comprised in the set of apparatuses:
- identifying, in the at least one analytical system 20 identified by the second identifier, a substitute apparatus 20PRE-2 that has an optical capability definition 32 enabling the visual identifier 3A to be decoded by the substitute apparatus 20PRE-2; and
- performing the at least one analytical test to be performed using the sample container 1 defined by the first identifier using the substitute apparatus 20PRE-2 of the at least one analytical system 20.

In this case, if an apparatus intended to be used to perform a workflow step of an analytical test is predicted in advance of being incapable of reading visual identifier reliably, the data processing agent 30SERV and/or the laboratory information system LIS search for a substitute apparatus within the analytical system 20 that is capable of reading the visual identifier reliably. The workflow for the chosen analytical test defined in the order data 34 is therefore updated with the substitute apparatus before execution of the workflow.

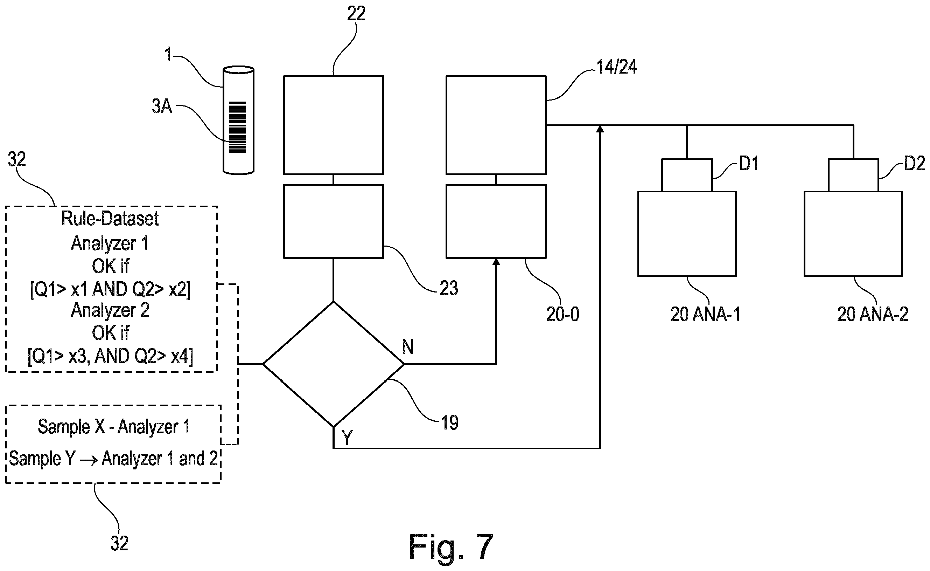

Fig. 7 schematically illustrates a further example of a rule-based approach for sample container examination.

According to an embodiment, comparing the capability definition 32 of the at least one apparatus to the digital representation of the visual identifier 3A further comprises:
obtaining, for the at least one apparatus comprised in the at least one analytical system 20, a rule set defining at least one rule of the capability definition 32 for the at least one apparatus that a visual identifier 3A associated with a sample container 1 should satisfy; and
if the visual identifier 3A associated with a sample container 1 satisfies all, or a predetermined subset, of the rules of the rule set, declaring that the visual identifier 3A is compatible with the at least one apparatus comprised in the at least one analytical system 20.

If the visual identifier 3A associated with a sample container 1 does not satisfy all, or the predetermined subset, of the rules of the rule set, declaring that the visual identifier 3A is not compatible with the at least one apparatus comprised in the at least one analytical system 20.

According to an example, the rule set may comprise one, or any combination, of a barcode edge determination metric, a barcode minimum reflectance metric, a barcode minimum edge contrast metric, a barcode symbol contrast metric, a barcode modulation grading, a barcode defects grading, and/or a barcode decodability grading.

Fig. 7 illustrates that for each sample container 1 associated with visual identifier 3A entering the analyser system 20, a visual identifier quality detection step 22 is performed, with quality metrics of the visual identifier extracted at step 23. A rule dataset as illustrated in data structure 32 of Fig. 6 provides, for each apparatus of the analytical system 20, a set of rules captured visual identifier can be acceptably decoded by a corresponding apparatus. A test order 34 associated with each sample maps the destination of each sample to one or more analysers within the analytical system 20. A rule checker 19 determines whether, or not, a visual identifier quality is compatible with an intended test order, based on the rules associated with the analysers comprised in a specific analyser system 20. If compatibility is determined, the sample container is forwarded to analysers downstream 20ANA-1, 20ANA-2, with a good degree of certainty that the optical identifier readers D1, D2 associated with those analysers can read the visual identifier 3A. If the rule tracker determines that the quality of the visual identifier 3A is not compatible with optical identifier readers D1, D2 of analysers downstream 20ANA-1, 20ANA-2, the relevant sample container 1 is sorted out of the set of sample container is provided to the laboratory for replacement of its visual identifier.

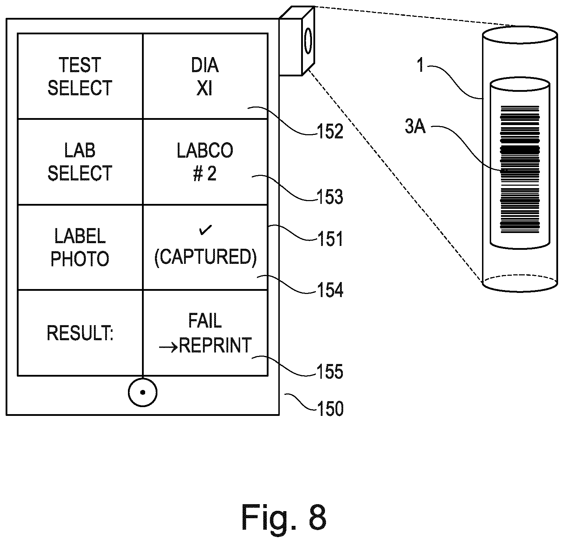

**Fig. 8** schematically illustrates an example of a graphical user interface during sample container examination.

In an exemplary setup, a specific test order and a lab analyzer config table are provided which define the used analyzer e.g., in form of a matrix. The combination of the used analyzer and a specific barcode reader capability info of the used analyzer define the barcode quality need e.g., in form of a matrix. The barcode quality need plus the specific barcode quality measured by the device used at the collection site define an OK or nOK for the lab processing. Accordingly, an error message or an ok is sent to the operator.

In the illustration of Fig. 8, a smart phone 150 usable the sample reception facility 10 is used to photograph a sample container 1 bearing a visual identifier 3A. First graphical user interface menu option "Test Select" enables a user to select, using a menu 152, a test for Diabetes indications "DIA". If the sample reception facility 10 have commercial arrangements with more than one laboratory, a specific laboratory intended to perform the test is selected using menu 153. Of course, the range of laboratories available at menu 153 may be amended based on the type of test chosen by menu 152. Graphical user interface option 154 prompts the user to upload an image of the identifier 3A and/or its mounting context on the sample container 1. Alternatively, or in addition, an optical identifier reader such as a barcode readeror QR code reader may be accessible to the user device. Therefore, the graphical user interface option 154 can also prompt the user to obtain and upload a visual identifier determination using the optical identifier reader. The software presents to the user the classification result in graphical user interface option 155. The result is derived by performing the method according to the first aspect. In a case where the visual identifier 3A is determined to be of too low quality to enable the sample container 1 to be entered into the analytical system 20, the graphical user interface may provide an option to reprint the visual identifier 3A, and alternatively or in addition provide advice as to the reasons that the previous visual identifier failed the determination according to the first aspect.

According to an embodiment, if the visual identifier associated with the sample container 1 can be decoded by the at least one apparatus of the analytical system 20, there is provided outputting a message confirming that the visual identifier associated with the sample container 1 has received a positive classification in respect of the at least one apparatus of the at least one analytical system 20.

According to an embodiment, if the visual identifier 3A associated with the sample container 1 cannot be decoded by the at least one apparatus 20PRE-1 of the analytical system 20, there is provided:
- generating user advice based on a comparison of the capability definition 32 of the at least one apparatus to the digital representation of the visual identifier 3A; and
- displaying the user advice via a user interface 151 of a user device 150.

According to an embodiment, if the visual identifier 3A associated with the sample container 1 cannot be decoded by the at least one apparatus 20PRE-1 of the analytical system 20, there is provided:
- printing a further label comprising the visual identifier 3A associated with the sample container 1 using a label printer 10Print, 20Print.

According to an embodiment, the method further comprises storing a plurality of digital representations of a visual identifier 3A associated with a corresponding plurality of sample containers; and
for each stored digital representation of a visual identifier 3A associated with a respective sample container:
- modelling a substitution of at least one apparatus comprised within the at least one analytical system 20 according to the second identifier, that will be used to perform the at least one analytical test according to the first identifier, with a substitute apparatus having a predetermined optical capability definition 32.

As illustrated in **Fig. 6****,** after a comparison stage 73 between apparatuses comprised in an analytical system 20 and a digital representation of a visual identifier obtained at the sample reception facility 10, at least one apparatus in the analytical system 20 may be considered to reliably identify the sample container bearing the visual identifier obtained at the sample reception facility 10. According to the present embodiment, the comparison stage 73 identifies at least one substitute apparatus in the analytical system 20 that can substitute for the workflow step defined by the order data 34. The comparison stage 73 models the substitution of the at least one substitute apparatus in the workflow step defined by the order data 34, so that the test defined by the order data 34 can still be performed by the analytical system 20 without replacement of a visual identifier at the sample reception facility 10. This approach both minimizes the amount of label reprinting that needs to be performed at the sample reception facility 10, but still enables reliable processing of the sample container associated with the visual identifier.

According to a second aspect, there is provided a system 60 comprising an apparatus comprising an optical identifier reader 12, 22 and/or a camera 14, 24 configured to obtain a digital representation of a visual identifier 3A associated with a sample container 1. The system further comprises an analytical system 20 comprising at least one apparatus 20PRE-1 configured to perform at least one analytical test, and a communications network 62. The system further comprises a data processing agent 30 that is communicably coupled to the apparatus and the analytical system 20 via the communications network.

The data processing agent 30 is configured to obtain a digital representation of a visual identifier 3A associated with a sample container 1, to identify at least one apparatus comprised within an analytical system 20, wherein the analytical system 20 is intended to perform at least one analytical test using the sample container 1.

The at least one apparatus 20PRE-1 comprises an optical identifier reader, wherein the data processing agent is further configured to classify the visual identifier 3A associated with the sample container 1 by characterizing the ability of the optical identifier reader of the at least one apparatus comprised in the analytical system 20 to readthe visual identifier 3A associated with the sample container 1, to thereby generate a corresponding classification result characterizing the visual identifier 3A associated with the sample container 1 to output a message defining the classification result.

According to an embodiment, the system further comprises a label printer 10Print, 20Print. The data processing agent 30 is configured to transmit a specification to the label printer 10Print, 20Print based on the order data 34. The label printer 10Print, 20Print is configured to print a replacement label for attachment to a sample container 1 according to the specification received from the data processing agent.

According to a fourth aspect, there is provided a computer program element comprising machine readable instructions which, when executed, performs the computer implemented method according to the first aspect.

According to a fifth aspect, there is provided a computer readable medium having encoded thereon the computer program element according to the fourth aspect.

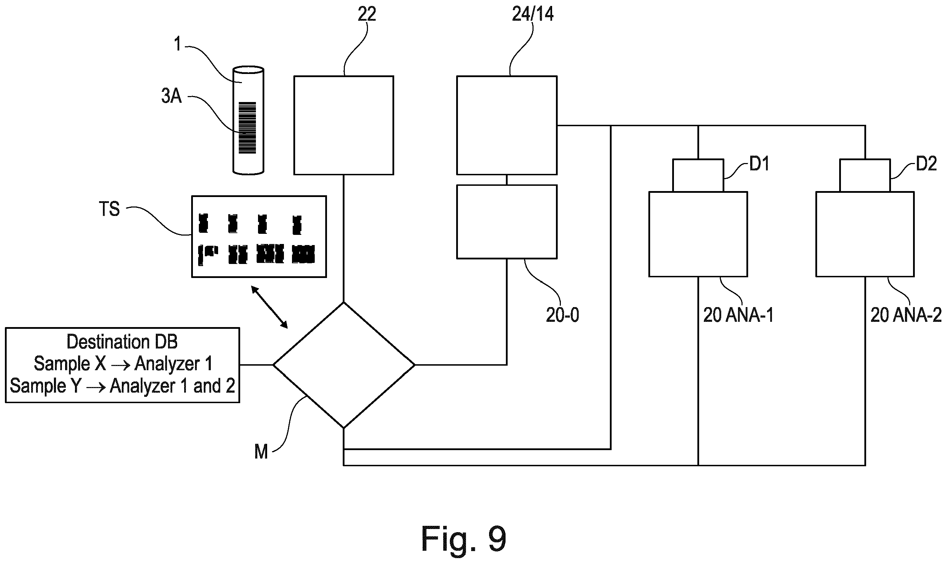

**Fig. 9** schematically illustrates an optional example of machine learning sample container classification.

According to a sixth aspect, there is provided a computer implemented method 80 for training a classifier of analytical sample containers comprising visual identifiers 3A, wherein the method comprises:
- obtaining a training set comprising a plurality of digital representations of visual identifiers 3A associated with a corresponding plurality of sample containers;
- labelling each digital representation in the training set with a first identifier of at least one analytical test to be performed using the sample container 1 and a second identifier of at least one analytical system 20 to be used to perform the at least one analytical test;
- obtaining a result set defining, for each digital representation in the training set, a determination of whether, or not, the corresponding visual identifier 3A was correctly read by all apparatuses of the at least one analytical system 20 and
- training, using a machine learning process, a classifier using the training set and the corresponding result set.

According to an embodiment, the computer implemented method 80 classifies the visual identifier associated with the sample container 1 based on the comparison between the capability definition 32 and the digital representation of the visual identifier is performed, at least partially, using the classifier trained according to the sixth aspect.

Therefore, besides providing a static (one generic threshold) lookup table of barcode compatibility of based on a one time measurement of optical identifier readers in the apparatuses, another implementation is optionally based on the continuous feedback and learning of the capability of optical indicator identifiers comprised in apparatuses in the analytical system 20. According to an embodiment, a machine learning algorithm is applied wherein the machine learning algorithm is trained on a dataset comprising digital representations of a plurality of sample containers, along with the success rate of optical indicator identifiers in the analytical system 20 at successfully classifying the visual identifiers associated with the sample containers. In particular, this enables the impact or drift of the capabilities as an optical identifier reader to be tracked over time as the ability of the optical identifier reader to readvisual identifiers changes due to environmental or ageing effects.

As shown in **Fig. 9****,** a training set TS comprising a variety of visual identifier s such as barcodes or QR codes is used to train a machine learning model M, typically by running a number of calibration sample containers comprising calibration identifiers through the analytical system 20, and recording, for each pair of calibration sample container and apparatus of the analytical system 20, whether or not a given sample container can be correctly decodedby the corresponding apparatus of the analytical system. A machine learning model M is trained on the pairs of data so obtained. The machine learning model M replaces, and/or supplements, the rule dataset stored in datastore 32, for example.

According to a seventh aspect, there is provided a computer program element comprising machine readable instructions which, when executed, performs the computer implemented method according to the fifth aspect.

According to an eighth aspect, there is provided a computer readable medium having encoded thereon the computer program element according to the seventh aspect.

According to a ninth aspect, there is provided a machine learning model comprising machine readable instructions which, when provided with an input data vector, generate an output data vector according to the classifier trained according to the sixth aspect.

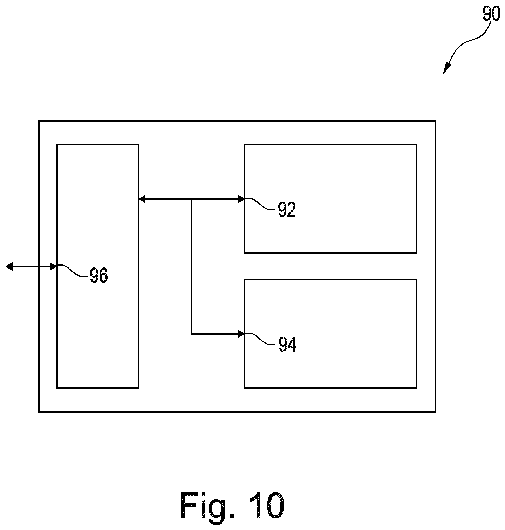

**Fig. 10** schematically illustrates an example of an apparatus according to the third aspect.

According to a third aspect, there is provided an apparatus 90 comprising a communications interface 96, a processor 92, and a memory interface 94.

The processor is configured to host a data processing agent that, in use, is communicably coupled to a apparatus and an analytical system 20 via a communications network. The data processing agent is configured to obtain a digital representation of a visual identifier 3A associated with a sample container 1, and to identify at least one apparatus comprised within the analytical system 20, wherein the analytical system 20 is intended to perform at least one analytical test using the sample container 1.

The data processing agent is further configured to classify the visual identifier 3A associated with the sample container 1 by characterizing the ability of the optical identifier reader of the at least one apparatus comprised in the analytical system 20 to readthe visual identifier 3A associated with the sample container 1, to thereby generate a corresponding classification result characterizing the visual identifier 3A associated with the sample container 1 to output a message defining the classification result.

Further disclosed is a computer program including computer-executable instructions for performing the method according to the present disclosure in one or more of the embodiments enclosed herein when the program is executed on a computer or computer network. Specifically, the computer program may be stored on a computer-readable data carrier. In other examples, the computer program can be Cloud-based computer programs. Thus, specifically, one, more than one or even all of method steps as disclosed herein may be performed by using a computer or a computer network, preferably by using a computer program.

Further disclosed is a computer program product having program code, in order to perform the method according to the present disclosure in one or more of the embodiments enclosed herein when the program is executed on a computer or computer network. Specifically, the program code may be stored on a computer-readable data carrier.

Further disclosed is a data carrier having a data structure stored thereon, which, after loading into a computer or computer network, such as into a working memory or main memory of the computer or computer network, may execute the method according to one or more of the embodiments disclosed herein.

Further disclosed is a computer program product with program code stored on a machine-readable carrier, in order to perform the method according to one or more of the embodiments disclosed herein, when the program is executed on a computer or computer network. Specifically, the computer program product may be distributed over a data network.

Further disclosed is a modulated data signal which contains instructions readable by a computer system or computer network, for performing the method according to one or more of the embodiments disclosed herein.

Further disclosed is a computer loadable data structure that is adapted to perform the method according to one of the embodiments described in this description while the data structure is being executed on a computer.

The features disclosed in the foregoing description, or the claims to follow, or in the drawings, whether described as apparatus features or in terms of means for performing a disclosed function, or in terms of method or process steps, may separately, or in any combination, used for providing the invention. While the invention has been described in terms of the embodiments of this description, a skilled person will be able to provide many equivalent modifications and variations based on this disclosure. Exemplary embodiments disclosed above are therefore for the purposes of illustration, and are not limiting. Changes to the embodiments described in this description can be provided without changing the spirit and scope of the invention. Paragraph headings introduced herein are not intended to limit the subject matter described. In this specification and the appended claims, the words "comprise" and "include", and their variations should be understood to imply the inclusion of a stated feature, or step or group of features, but not the exclusion of any other features.

### STATEMENTS

The invention is also defined according to the following embodiments:
A. A computer implemented method of an apparatus for analytical sample container classification, wherein the method comprises:
   - obtaining a digital representation of a visual identifier associated with a sample container;
   - obtaining order data comprising a first identifier of at least one analytical test or transfer action to be performed by an analytical system using the sample container, wherein the order data optionally comprises a second identifier of the at least one analytical system to be used to perform the at least one analytical test.
   - transmitting, via a communications network, the digital representation of the visual identifier to a data processing agent;
   - transmitting, via a communications network, the order data comprising the first identifier and optionally the second identifier to the data processing agent;
   - receiving, via a communications network, a message from the data processing agent defining a classification result defining a deciding metric characterizing an ability of the optical identifier reader of at least one apparatus comprised in the analytical system is capable of decoding a visual identifier associated with the sample container; and
   - outputting a message defining the classification result.
B. A computer implemented method of a data processing agent for analytical sample container classification , wherein the method comprises:
   - receiving, via a communications network coupled to an apparatus for analytical sample container classification, a digital representation of a visual identifier to a data processing agent;
   - receiving, from the apparatus, the order data comprising the first identifier and optionally the second identifier to the data processing agent;
   - identifying at least one apparatus comprised within an analytical system that will perform at least one analytical test using the sample container, wherein the at least one apparatus comprises an optical identifier reader;
   - classifying the sample container associated with the visual identifier based on a decoding metric characterizing the ability of the optical identifier reader of the at least one apparatus comprised in the analytical system is capable of decoding the visual identifier associated with the sample container, to thereby generate a corresponding classification result characterizing the sample container associated with the visual identifier; and
   - transmitting a message defining the classification result to the apparatus for analytical sample container classification.
C. An apparatus for analytical sample container classification, comprising:
   - a communications interface;
   - a processor;
   - an optical identifier reader; and
   - a user interface;

   wherein the optical identifier reader is configured to obtain a digital representation of a sample container associated with a visual identifier;
   wherein the processor is configured to transmit, via the communications interface, the digital representation of an visual identifier to a data processing agent, wherein the processor is configured to receive, from the data processing agent, a message defining a decoding metric characterizing the ability of the optical identifier reader of at least one apparatus comprised in the analytical system to decode a visual identifier associated with the sample container, and wherein the user interface is configured to output a message defining the classification result.
D. A data processing agent for analytical sample container classification, comprising:
   - a communications interface; and
   - a processor;

   wherein the communications interface is configured to receive from an apparatus for analytical sample container classification, a digital representation of a visual identifier to a data processing agent;
   wherein the processor is configured to identify at least one apparatus comprised within an analytical system intended to perform at least one analytical test using the sample container, wherein the at least one apparatus comprises an optical identifier reader, and to wherein the processor is configured to classify the visual identifier associated with the sample container based on a decoding metric characterizing the ability of the optical identifier reader of the at least one apparatus comprised in the analytical system to decode the visual identifier associated with the sample container, wherein the processor is further configured to generate a corresponding classification result characterizing the sample container associated with the visual identifier, and wherein the processor is further configured to transmit, via the communications interface, a message defining the classification result to the apparatus for analytical sample container classification.
E. A computer implemented method for analytical sample container classification, wherein the method comprises:
   - obtaining a digital representation of a visual identifier associated with a sample container;
   - identifying, using first and second identifiers, at least one apparatus comprised within the at least one analytical system that will be used to perform the at least one analytical test;
   - obtaining order data comprising the first identifier of at least one analytical test to be performed using the sample container, wherein the order data optionally further comprises the second identifier of at least one analytical system to be used to perform the at least one analytical test;
   - obtaining an optical capability definition of an optical identifier reader of the at least one apparatus;
   - comparing the optical capability definition of the at least one apparatus to the digital representation of the visual identifier; and
   - classifying the sample container associated with the visual identifier based on the comparison between the optical capability definition and the digital representation of the visual identifier.

## Claims

1. A computer implemented method (80) for analytical sample container classification, wherein the method comprises:
- obtaining (82) a digital representation of a visual identifier (3A) associated with a sample container (1);
- identifying (84) at least one apparatus (20PRE-1) comprised within an analytical system (20), wherein the analytical system (20) is intended to perform at least one analytical test using the sample container (1), wherein the at least one apparatus (20PRE-1) comprises an optical identifier reader;
- classifying (86) the sample container (1) associated with the visual identifier (3A) by characterizing the ability of the optical identifier reader and/or camera of the at least one apparatus (20PRE-1) comprised in the analytical system (20) to decode the visual identifier (3A) associated with the sample container (1), to thereby generate a corresponding classification result characterizing the sample container (1) associated with the visual identifier (3A); and
- outputting (88) a message defining the classification result.

2. The computer implemented method (80) according to claim 1, further comprising:
- obtaining a capability definition (32) of the optical identifier reader and/or camera of the at least one apparatus (20PRE-1) comprised in the analytical system (20), wherein the capability definition (32) characterizes at least one aspect of the optical identifier reader of the at least one apparatus (20PRE-1) to decode at least one visual identifier (3A); and
- classifying the visual identifier (3A) comprises comparing the capability definition (32) of the optical identifier reader of the at least one apparatus (20PRE-1) to the digital representation of the visual identifier (3A) associated with the sample container (1).

3. The computer implemented method (80) according to one of claims 1 or 2,
wherein identifying the at least one apparatus (20PRE-1) comprised within the analytical system (20) further comprises:
- obtaining order data comprising a first identifier of at least one analytical test to be performed by an analytical system (20) using the sample container (1), and/or at least one transfer action of the sample container (1) within the analytical system (20), wherein the order data optionally comprises a second identifier for identifying a specific analytical system (20) to be used to perform the at least one analytical test.

4. The computer implemented method (80) according to one of the preceding claims, further comprising:
if the sample container (1), and/or its associated visual identifier (3A) are classified with a negative classification result of the at least one apparatus (20PRE-1):
- outputting a message defining that the visual identifier (3A) associated with the sample container (1) has received a negative classification in respect of the at least one apparatus (20PRE-1), and/or
- outputting, to a laboratory information system associated with the at least one analytical system (20), a message comprising an identification code of a sample container (1) comprising the visual identifier (3A) that has received a negative classification in respect of the at least one apparatus (20PRE-1) comprised in the at least one analytical system (20).

5. The computer implemented method (80) according to one of claims 3 or 4,
wherein the at least one analytical system (20) comprises a set of apparatuses that will be used to perform the at least one analytical test in a predefined sequence defined by the analytical test identified by the first identifier of the order data (34); and further comprising:
- comparing a plurality of capability definitions (32) respectively corresponding to each apparatus (20PRE-1) of the set of apparatuses defined by the order data (34) with the digital representation of the visual identifier (3A); and
if the visual identifier (3A) associated with the sample container (1) does not meet the capability definition (32) of at least one apparatus (20PRE-1) comprised in the set of apparatuses:
- outputting a message defining that the visual identifier (3A) associated with the sample container (1) has received a negative classification in respect of the at least one apparatus (20PRE-1) comprised in the set of apparatuses, wherein the message optionally identifies each apparatus (20PRE-1) comprised in the set of apparatuses that triggered the negative classification.

6. The computer implemented method (80) according to one of claims 3 to 5, further comprising:
wherein the at least one analytical system (20) identified by the second identifier comprises a set of apparatuses that will be used to perform the at least one analytical test in a predefined sequence defined by the analytical test identified by the first identifier; and further comprising:
- comparing a plurality of capability definitions (32) respectively corresponding to each apparatus (20PRE-1) of the set of apparatuses with the digital representation of the visual identifier (3A); and
if the visual identifier (3A) associated with the sample container (1) does not meet the optical capability definition (32) of at least one apparatus (20PRE-1) comprised in the set of apparatuses:
- identifying, in the at least one analytical system (20) identified by the second identifier, a substitute apparatus (20PRE-2) that has an optical capability definition (32) enabling the visual identifier (3A) to be read by the substitute apparatus (20PRE-2); and
- performing the at least one analytical test to be performed using the sample container (1) defined by the first identifier using the substitute apparatus (20PRE-2) of the at least one analytical system (20).

7. The computer implemented method (80) according to one of claims 1 to 6, further comprising:
if the visual identifier (3A) associated with the sample container (1) cannot be decoded by the at least one apparatus (20PRE-1) of the analytical system (20):
- generating user advice based on a comparison of the capability definition (32) of the at least one apparatus to the digital representation of the visual identifier (3A); and
- displaying the user advice via a user interface (151) of a user device (150); and/or
- printing a further label comprising the visual identifier (3A) associated with the sample container (1) using a label printer (10Print, 20Print).

8. The computer implemented method (80) according to one of claims 2 to 7,
wherein comparing the capability definition (32) of the at least one apparatus to the digital representation of the visual identifier (3A) further comprises:
obtaining, for the at least one apparatus comprised in the at least one analytical system (20), a rule set defining at least one rule of the capability definition (32) for the at least one apparatus that a visual identifier (3A) associated with a sample container (1) should satisfy; and
if the visual identifier (3A) associated with a sample container (1) satisfies all, or a predetermined subset, of the rules of the rule set, declaring that the visual identifier (3A) is compatible with the at least one apparatus comprised in the at least one analytical system (20), or if the visual identifier (3A) associated with a sample container (1) does not satisfy all, or the predetermined subset, of the rules of the rule set, declaring that the visual identifier (3A) is not compatible with the at least one apparatus comprised in the at least one analytical system (20).

9. The computer implemented method (80) according to one of claims 2 to 8,
wherein the capability definition (32) of the optical identifier reader of the at least one apparatus characterizes at least one, or any combination, of a range of acceptable alignment deviations of the visual identifier relative to a longitudinal axis of a sample container; a range of acceptable occlusions of the visual identifier; a range of acceptable vertical and/or horizontal dimensions of the visual identifier; a range of acceptable blurring or resolution artefacts of the visual identifier; a range of acceptable contrast or brightness ratios of the visual identifier; and/or a range of acceptable artefacts of the visual identifier; and /or
wherein the capability definition (32) of the optical identifier reader of the at least one apparatus further characterizes at least one, or any combination, of a barcode edge determination metric, a barcode minimum reflectance metric, a barcode minimum edge contrast metric, a barcode symbol contrast metric, a barcode modulation grading, a barcode defects grading, and/or a barcode readability grading.

10. The computer implemented method (80) according to one of claims 3 to 9, further comprising:
- storing a plurality of digital representations of a visual identifier (3A) associated with a corresponding plurality of sample containers; and
for each stored digital representation of a visual identifier (3A) associated with a respective sample container:
- modelling a substitution of at least one apparatus comprised within the at least one analytical system (20) according to the second identifier that will be used to perform the at least one analytical test according to the first identifier, with a substitute apparatus having a predetermined optical capability definition (32); and optionally:
- outputting a corresponding identifier of one or more sample containers comprising a visual identifier (3A) that will receive a negative classification.

11. A computer implemented method (80) for training a classifier of analytical sample containers comprising visual identifiers (3A), wherein the method comprises:
- obtaining a training set comprising a plurality of digital representations of visual identifiers (3A) associated with a corresponding plurality of sample containers;
- labelling each digital representation in the training set with a first identifier of at least one analytical test to be performed using the sample container (1), and a second identifier of at least one analytical system (20) to be used to perform the at least one analytical test;
- obtaining a result set defining, for each digital representation in the training set, a determination of whether, or not, the corresponding visual identifier (3A) was correctly read by all apparatuses of the at least one analytical system (20); and
- training, using a machine learning process, a classifier using the training set and the corresponding result set.

12. A system (60) comprising:
- an apparatus comprising an optical identifier reader (12, 22) and/or a camera (14, 24) configured to obtain a digital representation of a visual identifier (3A) associated with a sample container (1);
- an analytical system (20) comprising at least one apparatus (20PRE-1) configured to perform at least one analytical test;
- a communications network (62); and
- a data processing agent (30) that is communicably coupled to the apparatus and the analytical system (20) via the communications network;
wherein the data processing agent (30) is configured to obtain a digital representation of a visual identifier (3A) associated with a sample container (1), to identify at least one apparatus comprised within an analytical system (20), wherein the analytical system (20) is intended to perform at least one analytical test using the sample container (1);
wherein the at least one apparatus (20PRE-1) comprises an optical identifier reader, wherein the data processing agent is further configured to classify the visual identifier (3A) associated with the sample container (1) by characterizing the ability of the optical identifier reader of the at least one apparatus comprised in the analytical system (20) to decode the visual identifier (3A) associated with the sample container (1), to thereby generate a corresponding classification result characterizing the visual identifier (3A) associated with the sample container (1) to output a message defining the classification result.

13. The system (60) according to claim 12, further comprising:
- a label printer (10Print, 20Print);
wherein the data processing agent (30) is configured to transmit a specification to the label printer (10Print, 20Print) based on the order data (34); and
wherein the label printer (10Print, 20Print) is configured to print a replacement label for attachment to a sample container (1) according to the specification received from the data processing agent.

14. A computer program element comprising machine readable instructions which, when executed, performs the computer implemented methods according to one of claims 1 to 10, or 11.

15. An apparatus (90) comprising:
- a communications interface (96);
- a processor (92); and
- a memory interface (94);
wherein the processor is configured to host a data processing agent that, in use, is communicably coupled to an apparatus and an analytical system (20) via a communications network;
wherein the data processing agent is configured to obtain a digital representation of a visual identifier (3A) associated with a sample container (1), and to identify at least one apparatus comprised within the analytical system (20), wherein the analytical system (20) is intended to perform at least one analytical test using the sample container (1);
wherein the data processing agent is further configured to classify the visual identifier (3A) associated with the sample container (1) by characterizing the ability of the optical identifier reader of the at least one apparatus comprised in the analytical system (20) to decode the visual identifier (3A) associated with the sample container (1), to thereby generate a corresponding classification result characterizing the visual identifier (3A) associated with the sample container (1) to output a message defining the classification result.
